# EUROPÄISCHE PATENTSCHRIFT

(11) **EP 0 620 851 B1**
(45) Veröffentlichungstag und Bekanntmachung des Hinweises auf die Patenterteilung: **15.10.1997**
(21) Anmeldenummer: 93901711.7
(22) Anmeldetag: 19.12.1992
(51) Int. Cl.: C12N 15/12, C12N 1/21, C12Q 1/68, C07K 14/435, G01N 33/50, G01N 33/577

(54) **NEUROBLASTOM-ASSOZIIERTES REGULATOR-GEN**
NEUROBLASTOMA-ASSOCIATED REGULATOR GENE
GENE REGULATEUR ASSOCIE AU NEUROBLASTOME

(30) Priorität: 23.12.1991 AT 2559/91
(43) Veröffentlichungstag der Anmeldung: 26.10.1994
(73) Patentinhaber: BOEHRINGER INGELHEIM INTERNATIONAL GmbH, 55218 Ingelheim am Rhein (DE); GENENTECH, INC., South San Francisco California 94080 (US)
(72) Erfinder: ELLMEIER, Wilfried, A-2340 Mödling (AT); WEITH, Andreas, A-1230 Wien (AT)
(74) Vertreter: Laudien, Dieter, Dr.
(86) Internationale Anmeldenummer: EP9202962
(87) Internationale Veröffentlichungsnummer: WO9313205

(56) Entgegenhaltungen:
- MOLCULAR AND CELLULAR BIOLOGY Bd. 10, Nr. 10, Oktober 1990, AM. SOC.MICROBIOL., WASHINGTON,DC,US; Seiten 5416 - 5423 R. VERSTEEG ET AL. 'N-myc expression switched off and class I human leucocyte antigen expression switched on after somatic cell fusion of neuroblastoma cells'
- PROC. NATL. ACAD SCI. Bd. 88, Nr. 5, 1. März 1991, NATL. ACAD SCI.,WASHINGTON, DC,US; Seiten 1815 - 1819 B.A.CHRISTY ET AL. 'An Id-related helix-loop-helix protein encoded by a growth factor-inducible gene' in der Anmeldung erwähnt
- GENES, CHROMOSOMES & CANCER Bd. 1, Nr. 1, September 1989, LISS,INC.,NY,US; Seiten 67-78 T. Martinsson et al.
- GENES, CHROMOSOMES & CANCER Bd. 1, Nr. 2, November 1989, LISS, INC.,NY,US; Seiten 159 - 166 A. WEITH ET AL. 'Neuroblastoma consensus deletion maps to 1p36.1-2' in der Anmeldung erwähnt
- PROC. NATL. ACAD SCI. Bd. 86, Nr. 10, Mai 1989, NATL. ACAD SCI.,WASHINGTON,DC, US; Seiten 3753 - 3757 C.-T. FONG ET AL. 'Loss of heterocygosity for the short arm of chromosome 1 in human neuroblastomas: Correlation with N-myc amplification' in der Anmeldung erwähnt
- EMBO JOURNAL Bd. 11, Nr. 7, Juli 1992, IRL PRESS LIM., OXFORD, ENGL.; Seiten 2563 - 2571 W. ELLMEIER ET AL. 'Mutually exclusive expression of a helix-loop- helix gene and N-myc in human neuroblastomas and in normal development'

### SEQUENZ PROTOKOLL

(1) ALLGEMEINE INFORMATION:
(ii) ANMELDETITEL: Neuroblastom-assoziiertes Regulatorgen
(iii) ANZAHL DER SEQUENZEN: 6
(iv) COMPUTER-LESBARE FORM:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0. Version #1.25 (EPA)

### (2) INFORMATION ZU SEQ ID NO: 1:

(i) SEQUENZ CHARAKTERISTIKA :
   (A) LÄNGE: 982 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 1:
   AGACAATTTT CAGCAGGAAG AAGTAGAAAG GATAAA ATG GAT CCT GCA CCA CGG 54
   Met Asp Pro Ala Pro Arg
   1 5
   GAA CCT CAC AGC ACC TCA CTT CTT TTG GTT TTC TTT CTC TTT GGG GCA 102
   Glu Pro His Ser Thr Ser Leu Leu Leu Val Phe Phe Leu Phe Gly Ala
   10 15 20
   CCT CTG GAC TCA CTC CCC AGC ATG AAG GCG CTG AGC CCG GTG CGC GGC 150
   Pro Leu Asp Ser Leu Pro Ser Met Lys Ala Leu Ser Pro Val Arg Gly
   25 30 35
   TGC TAC GAG GCG GTG TGC TGC CTG TCG GAA CGC AGT CTG GCC ATC GCC 198
   Cys Tyr Glu Ala Val Cys Cys Leu Ser Glu Arg Ser Leu Ala Ile Ala
   40 45 50
   CGG GGC CGA GGG AAG GGC CCG GCA GCT GAG GAG CCG CTG AGC TTG CTG 246
   Arg Gly Arg Gly Lys Gly Pro Ala Ala Glu Glu Pro Leu Ser Leu Leu
   55 60 65 70
   GAC GAC ATG AAC CAC TGC TAC TCC CGC CTG CGG GAA CTG GTA CCC GGA 294
   Asp Asp Met Asn His Cys Tyr Ser Arg Leu Arg Glu Leu Val Pro Gly
   75 80 85
   GTC CCG AGA GGC ACT CAG CTT AGC CAG GTG GAA ATC CTA CAG CGC GTC 342
   Val Pro Arg Gly Thr Gln Leu Ser Gln Val Glu Ile Leu Gln Arg Val
   90 95 100
   ATC GAC TAC ATT CTC GAC CTG CAG GTA GTC CTG GCC GAG CCA GCC CCT 390
   Ile Asp Tyr Ile Leu Asp Leu Gln Val Val Leu Ala Glu Pro Ala Pro
   105 110 115
   GGA CCC CCT GAT GGC CCC CAC CTT CCC ATC CAG ACA GCC GAG CTC GCT      438
   Gly Pro Pro Asp Gly Pro His Leu Pro Ile Gln Thr Ala Glu Leu Ala
       120                 125                 130
   CCG GAA CTT GTC ATC TCC AAC GAC AAA AGG AGC TTT TGC CAC T            481
   Pro Glu Leu Val Ile Ser Asn Asp Lys Arg Ser Phe Cys His
   135                 140                 145
   GACTCGGCCG TGTCCTGACA CCTCCAGAAC GCAGGTGCTG GCGCCCGTTC TGCCTGGGAC    541
   CCCGGGAACC TCTCCTGCCG GAAGCCGGAC GGCAGGGATG GGCCCCAACT TCGCCCTGCC    601
   CACTTGACTT CACCAAATCC CTTCCTGGAG ACTGAACCTG GTGCTCAGGA GCGAAGGACT    661
   GTGAACTTGT GGCCTGAAGA GCCAGAGCTA GCTCTGGCCA CCAGCTGGGC GACGTCACCC    721
   TGCTCCCACC CCACCCCAAG TTCTAAGGTC TTTTCAGAGC GTGGAGGTGT GGAAGGAGTG    781
   GCTGCTCTCC AAACTATGCC AAGGCGGCGG CAGAGCTGGT CTTCTGGTCT CCTTGGAGAA    841
   AGGTTCTGTT GCCCTGATTT ATGAACTCTA TAATAGAGTA TATAGGTTTT GTACCTTTTT    901
   TACAGGAAGG TGACTTTCTG TAACAATGCG ATGTATATTA AACTTTTTAT AAAAGTTAAC    961
   ATTTTGCATA ATAAACGATT T                                              982

### (2) INFORMATION ZU SEQ ID NO: 2:

(i) SEQUENZ CHARAKTERISTIKA:
   (A) LANGE: 148 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 2:
   Met Asp Pro Ala Pro Arg Glu Pro His Ser Thr Ser Leu Leu Leu Val
   1 5 10 15
   Phe Phe Leu Phe Gly Ala Pro Leu Asp Ser Leu Pro Ser Met Lys Ala
   20 25 30
   Leu Ser Pro Val Arg Gly Cys Tyr Glu Ala Val Cys Cys Leu Ser Glu
   35 40 45
   Arg Ser Leu Ala Ile Ala Arg Gly Arg Gly Lys Gly Pro Ala Ala Glu
   50 55 60
   Glu Pro Leu Ser Leu Leu Asp Asp Met Asn His Cys Tyr Ser Arg Leu
   65 70 75 80
   Arg Glu Leu Val Pro Gly Val Pro Arg Gly Thr Gln Leu Ser Gln Val
   85 90 95
   Glu Ile Leu Gln Arg Val Ile Asp Tyr Ile Leu Asp Leu Gln Val Val
   100 105 110
   Leu Ala Glu Pro Ala Pro Gly Pro Pro Asp Gly Pro His Leu Pro Ile
   115 120 125
   Gln Thr Ala Glu Leu Ala Pro Glu Leu Val Ile Ser Asn Asp Lys Arg
   130 135 140
   Ser Phe Cys His
   145

### (2) INFORMATION ZU SEQ ID NO: 3:

(i) SEQUENZ CHARAKTERISTIKA :
   (A) LÄNGE: 360 Basenpaare
   (B) ART: Nukleinsäure
   (C) STRANGFORM: Einzel
   (D) TOPOLOGIE: linear
(ii) ART DES MOLEKÜLS: cDNS
(xi) SEQUENZBESCHREIBUNG: SEQ ID NO: 3:
   ATG AAG GCG CTG AGC CCG GTG CGC GGC TGC TAC GAG GCG GTG TGC TGC        48
   Met Lys Ala Leu Ser Pro Val Arg Gly Cys Tyr Glu Ala Val Cys Cys
     1               5                  10                  15
   CTG TCG GAA CGC AGT CTG GCC ATC GCC CGG GGC CGA GGG AAG GGC CCG        96
   Leu Ser Glu Arg Ser Leu Ala Ile Ala Arg Gly Arg Gly Lys Gly Pro
                20                  25                  30
   GCA GCT GAG GAG CCG CTG AGC TTG CTG GAC GAC ATG AAC CAC TGC TAC       144
   Ala Ala Glu Glu Pro Leu Ser Leu Leu Asp Asp Met Asn His Cys Tyr
            35                  40                  45
   TCC CGC CTG CGG GAA CTG GTA CCC GGA GTC CCG AGA GGC ACT CAG CTT       192
   Ser Arg Leu Arg Glu Leu Val Pro Gly Val Pro Arg Gly Thr Gln Leu
        50                  55                  60
   AGC CAG GTG GAA ATC CTA CAG CGC GTC ATC GAC TAC ATT CTC GAC CTG       240
   Ser Gln Val Glu Ile Leu Gln Arg Val Ile Asp Tyr Ile Leu Asp Leu
    65                  70                  75                  80
   CAG GTA GTC CTG GCC GAG CCA GCC CCT GGA CCC CCT GAT GGC CCC CAC       288
   Gln Val Val Leu Ala Glu Pro Ala Pro Gly Pro Pro Asp Gly Pro His
                    85                  90                  95
   CTT CCC ATC CAG ACA GCC GAG CTC GCT CCG GAA CTT GTC ATC TCC AAC       336
   Leu Pro Ile Gln Thr Ala Glu Leu Ala Pro Glu Leu Val Ile Ser Asn
               100                 105                 110
   GAC AAA AGG AGC TTT TGC CAC T GA                                      360
   Asp Lys Arg Ser Phe Cys His
           115

### (2) INFORMATION ZU SEQ ID NO: 4:

(i) SEQUENZ CHARAKTERISTIKA:
   (A) LÄNGE: 119 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 4:
   Met Lys Ala Leu Ser Pro Val Arg Gly Cys Tyr Glu Ala Val Cys Cys
     1               5                  10                  15
   Leu Ser Glu Arg Ser Leu Ala Ile Ala Arg Gly Arg Gly Lys Gly Pro
                20                  25                  30
   Ala Ala Glu Glu Pro Leu Ser Leu Leu Asp Asp Met Asn His Cys Tyr
            35                  40                  45
   Ser Arg Leu Arg Glu Leu Val Pro Gly Val Pro Arg Gly Thr Gln Leu
        50                  55                  60
   Ser Gln Val Glu Ile Leu Gln Arg Val Ile Asp Tyr Ile Leu Asp Leu
    65                  70                  75                  80
   Gln Val Val Leu Ala Glu Pro Ala Pro Gly Pro Pro Asp Gly Pro His
                    85                  90                  95
   Leu Pro Ile Gln Thr Ala Glu Leu Ala Pro Glu Leu Val Ile Ser Asn
               100                 105                 110
   Asp Lys Arg Ser Phe Cys His
           115

### (2) INFORMATION ZU SEQ ID NO: 5:

(i) SEQUENZ CHARAKTERISTIKA:
   (A) LÄNGE: 446 Basenpaare
   (B) ART: Nukleinsäure
   (C) STRANGFORM: Einzel
   (D) TOPOLOGIE: linear
(ii) ART DES MOLEKÜLS: DNS (genomisch)
(xi) SEQUENZBESCHREIBUNG: SEQ ID NO: 5:
   TTTATGACCT CGGAGGAGCT GTGGGCTCGA ACCAGTGTTG GGCTAAAGGC GACTGGCAGG        60
   GGGCAGGGAA GCTCAAAGAT CTGGGGTGCT GCCAGGAAAA AGCAAATTCT GAAAGTTAAT 120
   GGTTTTGAGT GATTTTTAAA TCCTTGCTGC CGGAGAGACC CACCTCTCCC CGGTATCAGC 180
   ACTTCCTCAT TCTTTGTATC CACGGCTCCG CGGTCTTCGG CGTCAGACCA GCCGGAGGAA 240
   GCCTGTTTGC AATTTAAGCG GGCTGTGTAC ACCCAGGGCC GACGGGGGCG GGGCCGAGGG 300
   CGGGCCATTT TGAATAAAGA GGCGTGCCTT CCAGGCAGGC TCTATAAGTG ACCGCCGCGG 360
   GCACGTGCGC CGTGCAGGTC ACTGTAGCGG GACTTCTTTT GGTTTTCTTT CTCTTTGGGG 420
   CACCTCTGGA CTCACTCCCC AGCATG 446

### (2) INFORMATION ZU SEQ ID NO: 6:

(i) SEQUENZ CHARAKTERISTIKA:
   (A) LÄNGE: 18 Basenpaare
   (B) ART: Nukleinsäure
   (C) STRANGFORM: Einzel
   (D) TOPOLOGIE: linear
(ii) ART DES MOLEKÜLS: synthetisches Oligodesoxyribonukleotid
(xi) SEQUENZBESCHREIBUNG: SEQ ID NO: 6:
   TGGGGAGTGA GTCCAGAG 18

## Beschreibung

Die Erfindung bezieht sich auf ein Gen, das signifikant von Tumor-spezifischen Deletionen in humanen Neuroblastomen betroffen und an der Tumorgenese beteiligt ist.

Einer Gruppe von Proteinen, von denen gezeigt wurde, daß sie vorwiegend als Aktivatoren der Transkription fungieren, sind Motive für die Dimerisierung und DNA-Bindung gemeinsam (Jones, 1990). Die Dimerisierungsdomäne ist eine amphipatische Helix-Loop-Helix (HLH)-Region, die DNA-Bindung wird durch einen Abschnitt basischer Aminosäuren, die der HLH-Domäne vorangestellt sind (Murre et al., 1989a,b), erleichtert. Proteine, die dieses basische Helix-Loop-Helix-Motiv (bHLH) enthalten, können Homo- und Heterodimere bilden. Die Modulation der transkriptionellen Aktivierung durch bHLH-Faktoren wird durch eine andere Gruppe von dimerisierenden Proteinen bewirkt. Dies wurde im Rahmen der Identifizierung des Id-Proteins festgestellt, eines Proteins, das ebenfalls ein Helix-Loop-Helix-Motiv enthält, dem jedoch die basische Region fehlt (HLH-Proteine) (Benezra et al., 1990). Id bildet Heterodimere mit einigen Mitgliedern aus der Familie der bHLH-Transkriptionsfaktoren. Da ihm die basische Region fehlt, die für die DNA-Bindung verantwortlich ist, können Heterodimere, die Id enthalten, nicht DNA binden. Daher werden bHLH-Proteine höchstwahrscheinlich durch HLH-Proteine, wie z.B. Id, negativ reguliert. Weitere Beispiele für regulatorische bHLH- und HLH-Proteinwechselwirkungen sind die Gene des Drosophila achaete-scute-Komplexes und das *extramacrochaetae* (*emc*)-Gen (Ellis et al., 1990; Garell und Modolell, 1990). Diese Gene haben eine Funktion bei der Entwicklung der Sinnesorgane des peripheren Nervensystems (Ghysen und Dambly-Chaudiere, 1989). Im allgemeinen wird von Genen, die für Helix-Loop-Helix-Proteine kodieren, vermutet, daß sie an der Kontrolle der Zelldifferenzierung beteiligt sind.

Die Zelldifferenzierung ist einer der Vorgänge, der durch die neoplastische Transformation beeinträchtigt wird. Daher dürfte die Funktion von Genen, die an diesen Prozessen beteiligt sind, in Tumorzellen gestört sein. Das Auftreten von sowohl dominanten wie rezessiven Mutationen in Tumoren dürfte die normale Funktion der betroffenen Gene als entweder positive oder negative Regulatoren widerspiegeln. Von Helix-Loop-Helix-Proteinen wurden bis jetzt ausschließlich Auswirkungen aufgrund dominanter Mutationen festgestellt, z.B. die oncogenen Aktivierungen von myc-Genen (Lüscher und Eisenmann, 1990; Zimmerman und Alt, 1990). Jedoch könnte aufgrund der verschiedenen Funktionen der für HLH-Proteine kodierenden Gene auch in Betracht gezogen werden, daß Gene dieses Typs von rezessiven Mutationen betroffen sind und daher Eigenschaften aufweisen, die Tumor-Suppressor-Genen entsprechen.

Allelische Deletionen in spezifischen Regionen des Genoms, die signifikant häufig in Tumor-Genomen auftreten, werden als Markierungspunkte für die Lage von Tumor-Suppressor-Genen (Weinberg, 1991) herangezogen. In Untersuchungen von humanen Neuroblastomen wurde eine Consensusdeletion im Chromosom lp36.2-p36.1 definiert (Weith et al., 1989). Der allelische Verlust dieses Abschnitts in ca. 80 bis 90 % der untersuchten Tumoren führte zu der Vermutung, daß ein Gen, das Tumore verhindert, in dieser Region gelegen sei.

Der vorliegenden Erfindung lag die Aufgabe zugrunde, ein solches Gen zu isolieren.

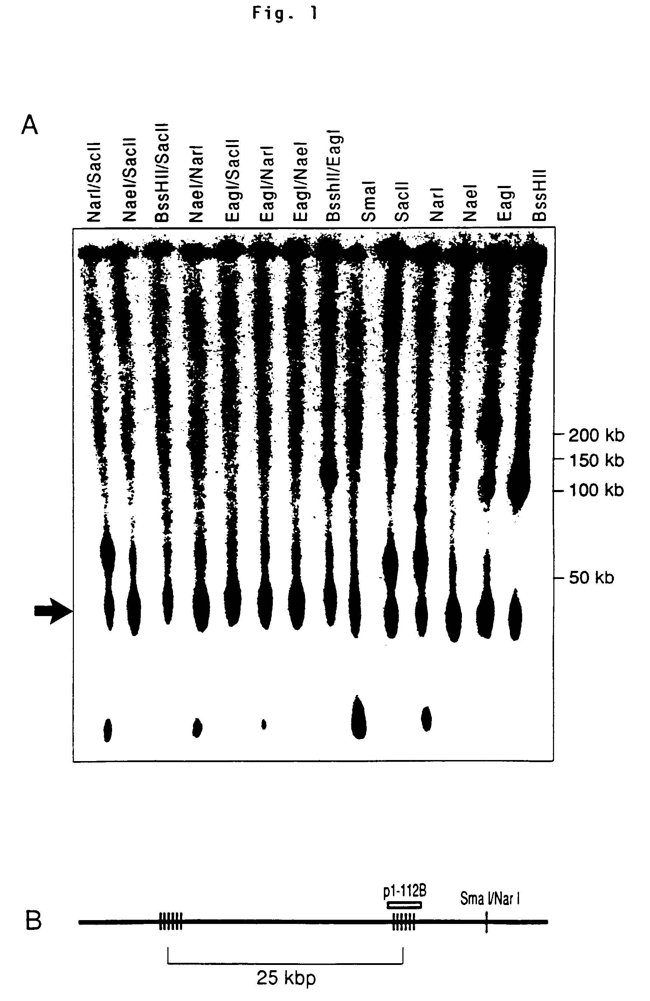

Zur Lösung der gestellten Aufgabe wurde wie folgt vorgegangen: Um Gene in der humanen Chromosom 1p36-Region zu identifizieren, wurde eine Palette von 1p36-spezifischen microklonierten DNA-Sonden (Martinsson et al., 1989) eingesetzt, um CpG-Islands zu identifizieren. CpG-Islands repräsentieren CpG-reiche, 0.5 - 1 kbp lange DNA-Abschnitte, die mit den 5'-Enden vieler Gene assoziiert sind (Bird, 1986). Die Cytosin-Reste in den Islands sind nicht methyliert, daher werden entsprechende Sequenzen von Methylierungsempfindlichen, selten schneidenden Restriktionsenzymen erkannt. Daher wurden einzelne Sonden mit Pulsfeld-Elektrophoreseblots von genomischer DNA hybridisiert, die entweder einfachem oder doppeltem Verdau mit selten schneidenden Restriktionsenzymen unterworfen worden war. Von jenen DNA-Sonden, die zwischen zwei CpG-Islands kartiert wurden, wurde erwartet, daß sie mit gleich großen Fragmenten in verschiedenen Einzel- und Doppelverdauen hybridisieren würden. Die Sonde mit der Bezeichnung pl-112B, kartiert in lpter-p36.12, zeigte eine Bande von 25 kb in Bss HII-, Eag I-, Nae I-, Nar I-, Sac II- und Sma I-Verdauen (Fig.lA). Die Bande behielt dieselbe Größe in DNA, die mit Kombinationen dieser Enzyme verdaut worden war. Daher kartiert pl-112B offensichtlich in einem 25 kb-Abschnitt zwischen zwei Clustern von Erkennungsstellen für selten schneidende Restriktionsenzyme (Fig. 1B).

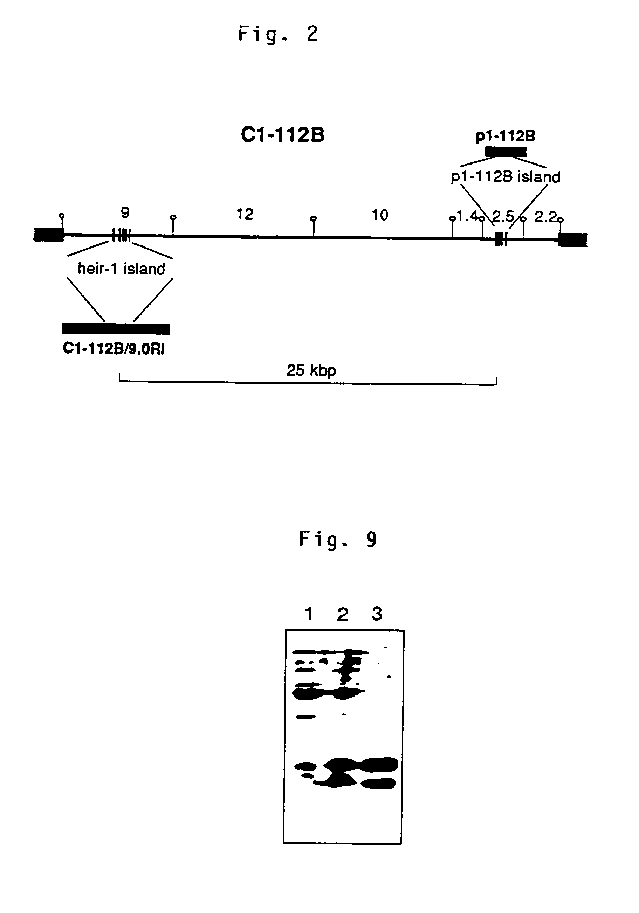

Um diese Cluster zu klonieren, wurde eine genomische Cosmid-Library mit p1-112B durchsucht und ein positiver Klon isoliert. Das Cosmid, das mit C1-112B bezeichnet wurde, enthielt ein 37 kb genomisches DNA-Insert. Restriktionskartierung dieses Klons mit selten schneideneden Restriktionsenzymen ergab zwei Cluster von Restriktionsschnittstellen im Abstand von ungefähr 25 kb zueinander (Fig. 2). Von einem der Cluster zeigte sich, daß es in der p1-112B-Sonde gelegen ist. Das andere Cluster wurde in ein 9.0 kb-EcoRI-Fragment subkloniert (C1-112B/9.ORI). Die Hybridisierung von C1-112B/9.ORI auf Southern Blots mit DNA, die mit den Enzymen aus den Clustern geschnitten worden war, zeigte, daß die seltenen Schnittstellen in der genomischen DNA offensichtlich nicht methyliert sind und daß das Cluster von seltenen Schnittstellen ein CpG-Island darstellt. Ähnliche Ergebnisse wurden für das Cluster erhalten, das in der p1-112B-Sonde enthalten ist.

CpG-Islands stellen das 5'-Ende von Genen dar und schließen oft einen Teil der ersten Exons mit ein (Bird, 1986). Um zu bestimmen, ob C1-112B/9.ORI ein Transkript erkennt, wurde ein Northern Blot von Gesamt-RNA aus HeLa-Zellen mit der Sonde untersucht. Eine positive Bande wurde bei ca. 1.2 kb nachgewiesen. Daraufhin wurde eine HeLa-cDNA-Library mit C1-112B/9.ORI durchsucht. Zwei verschiedene cDNA-Klone verschiedener Länge wurden isoliert. Die beiden Klone wurden sequenziert; die Sequenzanalyse ergab eine identische Sequenz beider Klone, abgesehen davon, daß dem kürzeren Klon 200 bp am 5'-Ende fehlten. Der längere Klon wurde mit HEIR-1 bezeichnet und für die weiteren Versuche verwendet. Seine Sequenz (SEQ ID NO:1) umfaßt 982 Nukleotide und enthält einen einzigen offenen Leserahmen, beginnend mit einem Startcodon bei Position 37 und endend mit einem TGA-Stopcodon bei Position 481. Im Leserahmen mit dem ersten ATG wurde bei Position 124 ein zusätzliches mögliches Startcodon festgestellt. Die 3'-nicht-translatierte Region enthält ein poly(A)-Signal bei Position 971. Ein ATTTA-Motiv, von dem gezeigt wurde, daß es für die kurze / Halbwertszeit der mRNA verantwortlich ist (Brawerman, 1989), wurde ebenfalls in einem AT-reichen Abschnitt des 3'-nicht-translatierten Teils der Message gefunden.

Da im Leserahmen 5' vom ersten Start-Codon kein Stop-Codon gefunden wurde, wurde zunächst nicht ausgeschlossen, daß sich der Leserahmen weiter in 5'-Richtung erstreckt.

Direkte Sequenzierung der Region 5' vom ATG-Codon an Position 124 aus dem Cosmid C1-112B zeigte, daß die Sequenz des HEIR-1 cDNA-Klons in diesem Abschnitt von der genomischen DNA abweicht. Direkte Sequenzanalyse der 5'-Region (Nukleotid 1-123) aus dem cDNA-Klon ergab keine Homologie zu dem genomischen Lokus. Es wurde daher angenommen, daß dieser DNA-Abschnitt des HEIR-1 cDNA-Klons einen Klonierungsartefakt darstellt.

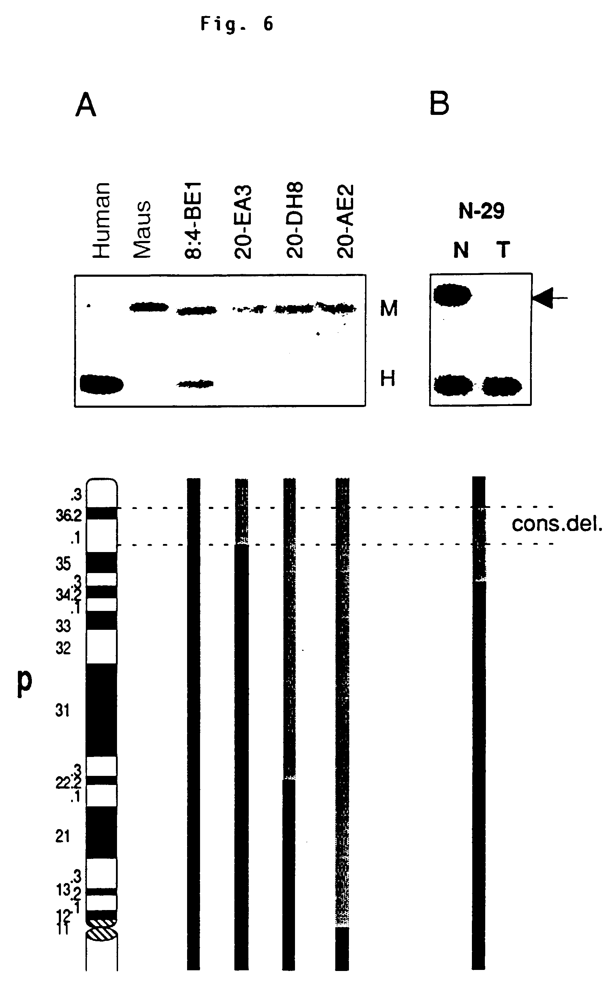

Das genomische DNA-Fragment p1-112B liegt auf Chromosom lpter-p36.12. Um die Lage des HEIR-1-Gens zu bestätigen, wurde eine Reihe von (HumanXMaus)-Microcell-Hybriden (Martinsson et al., 1989) für das Kartieren mittels Southern Blot (Southern Mapping) verwendet. Die Hybrid-Zellinien enthalten jeweils das humane Chromosom 1 mit verschiedenen Deletionen des 1p-Armes als einziges Humanmaterial auf einem Maus-Background. Die HEIR-1-cDNA wurde auf einem Southern Blot, enthaltend EcoRI-verdaute DNA der vier Hybridzellinien, getestet (Fig. 6). Ein positives humanes Fragment wurde nur in der DNA desjenigen Hybrids gefunden, das ein intaktes Chromosom 1 enthält, aber in keinem der Hybride, die Deletionen in der lpter-p36.12-Region aufwiesen. Daraus konnte gefolgert werden, daß HEIR-1 in derselben Chromosomenregion gelegen ist wie die genomische Sonde, die anfänglich für die Identifizierung des CpG-Islands verwendet worden war und daß es sich somit bei dem gefundenen Gen um das gesuchte handelt.

Der Deletions-Bruchpunkt der Hybrid-Zellinie 20-EA3 kennzeichnet die proximale Grenze der Neuroblastom-Consensus-Deletion (Martinsson et al., 1989; Weith et al., 1989). Da HEIR-1 distal von diesem Bruchpunkt lokalisiert ist, wurde außerdem überprüft, ob der Genlocus innerhalb der Grenzen der Consensus-Deletion kartiert. Zu diesem Zweck wurde ein Neuroblastom-Tumor mit der Bezeichnung N-29 (Weith et al., 1989) mit einer allelischen Deletion untersucht, deren distaler Bruchpunkt die distale Grenze der Consensus-Deletion markiert. Unter Verwendung eines Restriktionsfragment-Längenpolymorphismus (RFLP) wurde eine allelische Deletion im HEIR-1-Lokus dieses Tumors nachgewisen. Dies war der Beweis dafür, daß HEIR-1 in der Neuroblastom-Consensus-Deletion kartiert.

Die vorliegende Erfindung betrifft eine neue humane DNA, die in der Neuroblastom-Consensusdeletion 1p36.2-p36.1 kartiert und die für ein Helix-Loop-Helix-Protein der Bezeichnung HEIR-1 kodierende Region enthält.

In einem Aspekt der Erfindung ist die DNA genomische DNA.

In einem weiteren Aspekt der Erfindung ist die DNA eine cDNA.

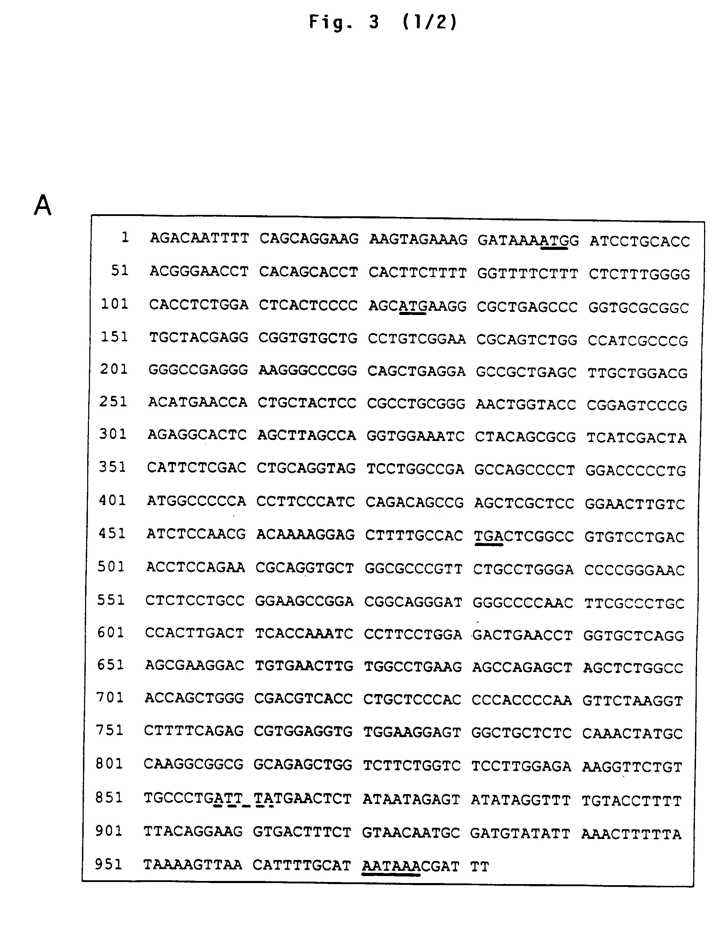

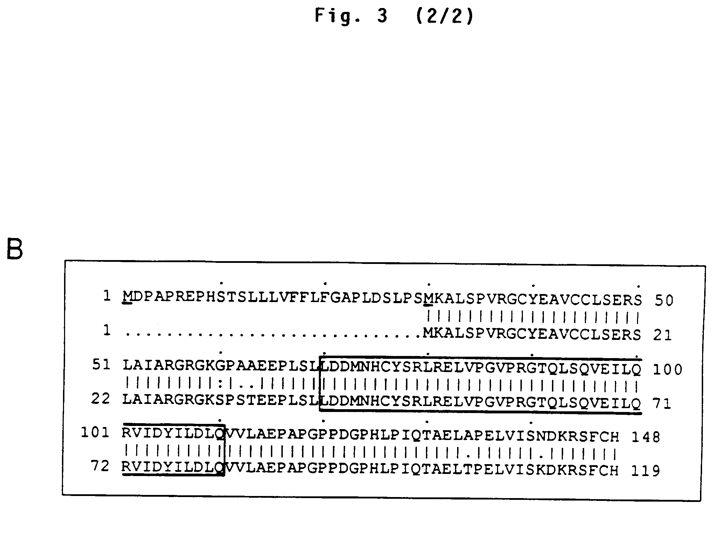

Der offene Leserahmen der HEIR-1-cDNA kodiert für ein Protein von 119 Aminosäuren, das ein Helix-Loop-Helix-Motiv enthält. Die kodierende Region der cDNA und die abgeleitete Polypetidsequenz sind als SEQ ID NO:3 und SEQ ID NO:4 dargestellt. (Der im isolierten cDNA-Klon, der die in SEQ ID NO:1 dargestellte Sequenz aufweist, ermittelte offene Leserahmen würde für ein Polypeptid von 148 Aminosäuren kodieren; die entsprechend abgeleitete Sequenz ist in SEQ ID NO:2 dargestellt.) Der Sequenzvergleich mit Mitgliedern der Familie der HLH-Transkriptionsfaktoren ergab eine Identität von 95.8 % mit dem Maus-HLH462 (Christy et al., 1991) über die gesamten 119 Aminosäuren von HLH462 (Fig. 3B). Die Aminosäureunterschiede zwischen HEIR-1 und dem Maus-HLH462-Protein ergeben sich durch einen Austausch von fünf nicht-polaren durch polare Aminosäuren. Diese Übergänge liegen außerhalb des HLH-Motivs. Die N-terminalen 29 Aminosäuren des aus dem cDNA-Klon abgeleiteten Proteins sind im Maus-Protein nicht enthalten (Fig. 3B). Es wurde daher angenommen, daß das zweite ATG bei Position 124 mit dem Startcodon von HLH462 übereinstimmt, also daß das erste ATG überlesen wird und das zweite ATG das richtige Startcodon von HEIR-1 ist. Diese Annahme wurde durch die direkte Sequenzierung des genomischen Lokus (vgl. oben) bestätigt.

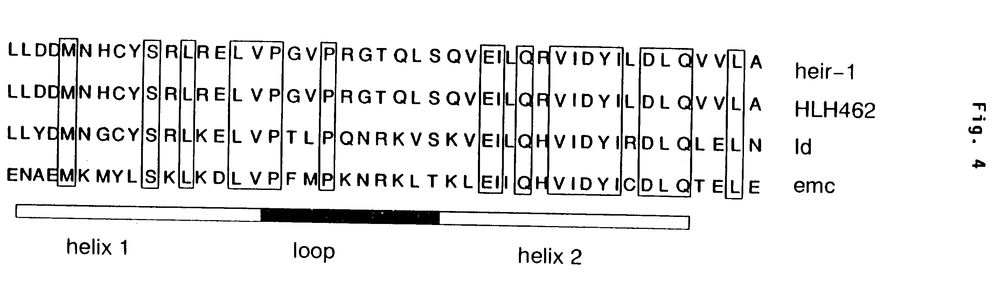

Das HLH-Motiv von HEIR-1, ebenso wie das von HLH462 (Christy et al., 1991), zeigt eine deutliche Homologie mit den HLH-Motiven von Id und dem Drosophila emc-Protein. Insbesondere sind 11 der 16 Aminosäuren der zweiten Helix zwischen HEIR-1, Id und emc konserviert (Fig. 4). Wie Id, *emc* und HLH462 fehlt HEIR-1 eine basische Region, die für die spezifische DNA-Bindung erforderlich ist und in den bHLH-Proteinen gefunden wurde. Es wurde jedoch eine deutliche Ähnlichkeit auch mit den HLH-Motiven von bHLH-Proteinen, wie MyoD (Davis et al., 1987), c-myc (Bernard et al., 1983) und E47 (Murre et al., 1989a) festgestellt.

Die Verfügbarkeit der HEIR-1-cDNA stellt die Voraussetzung dar, HEIR-1 in größeren Mengen als rekombinantes Protein herzustellen. Die Herstellung erfolgt in geeigneten prokaryotischen oder eukaryotischen Wirtsorganismen, z.B. E.coli, Hefe oder Zellen höherer Organismen. Die für die Produktion rekombinanter Polypeptide verwendeten Techniken sind dem Durchschnittsfachmann geläufig; für den jeweiligen Expressionswirt geeignete Vektoren, Kontrollsequenzen etc. können einschlägigen Handbüchern (z.B. Sambrook et al., 1989, Molecular Cloning A Laboratory Manual. Cold Spring Harbor Laboratory) entnommen werden.

Gegenstand der vorliegenden Erfindung sind somit in einem weiteren Aspekt rekombinante DNA-Moleküle, enthaltend die für HEIR-1 kodierende DNA sowie damit funktionell verbundene Expressionskontrollsequenzen, sowie die damit transformierten Wirtsorganismen.

Um festzustellen, ob in den isolierten Cosmidklonen das komplette HEIR-1-Gen enthalten ist, wird die Methode der Restriktionskartierung angewendet. Dazu wird Cosmid-DNA mit Restriktionsenzymen geschnitten, gelfraktioniert, auf Membranen transferiert und mit der HEIR-1-cDNA hybridisiert. Der Nachweis der Vollständigkeit ist gegeben, wenn die Cosmid-DNA alle Restriktionsfragmente aufweist, die der cDNA homolog sind. Weitere genomische Klone können aus den genomischen Libraries durch Screenen mit der erfindungsgemäßen cDNA erhalten werden. Der 5'-regulatorische Bereich wurde durch Sequenzierung mit Hilfe eines Primers ermittelt, der komplementär zur 5'-Region des kodierenden Genabschnittes war. Die Sequenzierung wurde in dem genomischen DNA Klon C1-112B/9.ORI vorgenommen. Die Sequenz, einschließlich dem Startcodon, ist in SEQ ID NO:5 dargestellt.

Gegenstand der vorliegenden Erfindung ist in einem weiteren Aspekt das rekombinante HEIR-1-Protein.

Die Verfügbarkeit des Proteins ermöglicht die Herstellung von Anti-HEIR-1-Antikörpern, vorzugsweise monoklonaler Anti-HEIR-1-Antikörper.

Die vorliegende Erfindung betrifft somit in einem weiteren Aspekt Antikörper gegen HEIR-1, vorzugsweise monoklonale Antikörper, sowie Hybridomzellen, die solche Antikörper produzieren, und Verfahren zu ihrer Herstellung.

Die Herstellung monoklonaler Antikörper ist dem Fachmann geläufig; geeignete Vorschriften können einschlägigen Handbüchern entnommen werden (z.B. Harlow and Lane, 1988). Die Herstellung beruht im wesentlichen auf der von Köhler und Milstein, 1975, beschriebenen Mehtode, nach der Tiere, z.B. Mäuse, mit dem Antigen immunisiert, B-Lymphozyten aus den immunisierten Tieren mit Myelomzellen fusioniert und aus den erhaltenen Hybridomen die Antikörper gewonnen werden.

Da das HEIR-1-Gen in einer genomischen Region kartiert, die häufig von allelischen Deletionen in Neuroblastomen betroffen ist, wurde eine Reihe von Neuroblastomen auf Deletionen des HEIR-1-Locus untersucht. Unter Verwendung eines Apa I-Restriktionsfragment-Längenpolymorphismus (RFLP)-Tests für diesen Locus wurde eine sog. LOH (loss of heterozygosity)-Analyse durchgeführt. Southern Blots, hergestellt mit DNAs aus sechzehn verschiedenen Neuroblastomen und den DNAs aus dem jeweils entsprechenden Normalgewebe, verdaut mit Apa I, wurden mit HEIR-1-cDNA als Sonde untersucht.

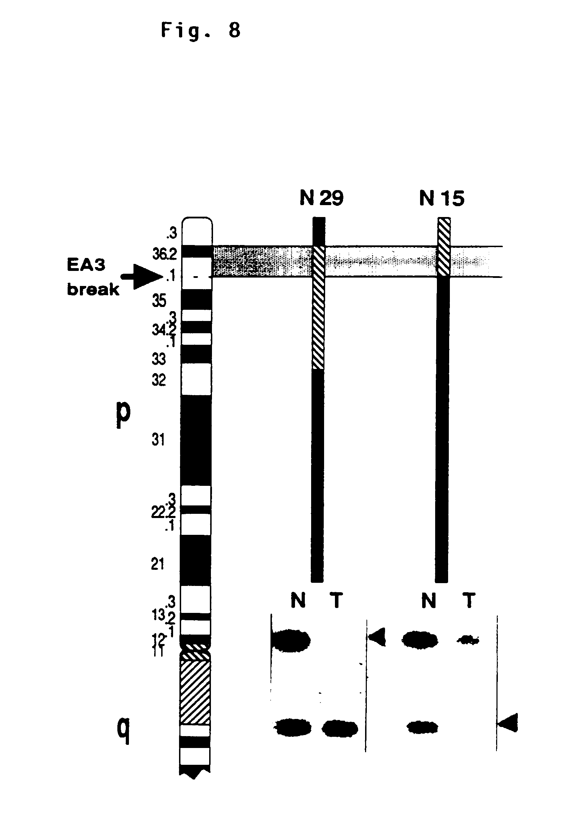

Heterozygotie des Locus wurde in der DNA von elf verschiedenen Normalgeweben festgestellt. Von diesen elf Fällen zeigten sieben einen tumorspezifischen Verlust eines Allels. Unter diesen sieben Tumoren waren zwei, von denen allelische Deletionen nachgewiesen worden waren, die die proximale bzw. die distale Grenze der Neuroblastom-Consensusdeletion darstellen (Fig. 8) (Weith et al., 1989). Da die HEIR-1-Sonde in beiden Tumoren einen Verlust der Heterozygotie zeigte, war der Locus offensichtlich der Consensusdeletion zuzuordnen.

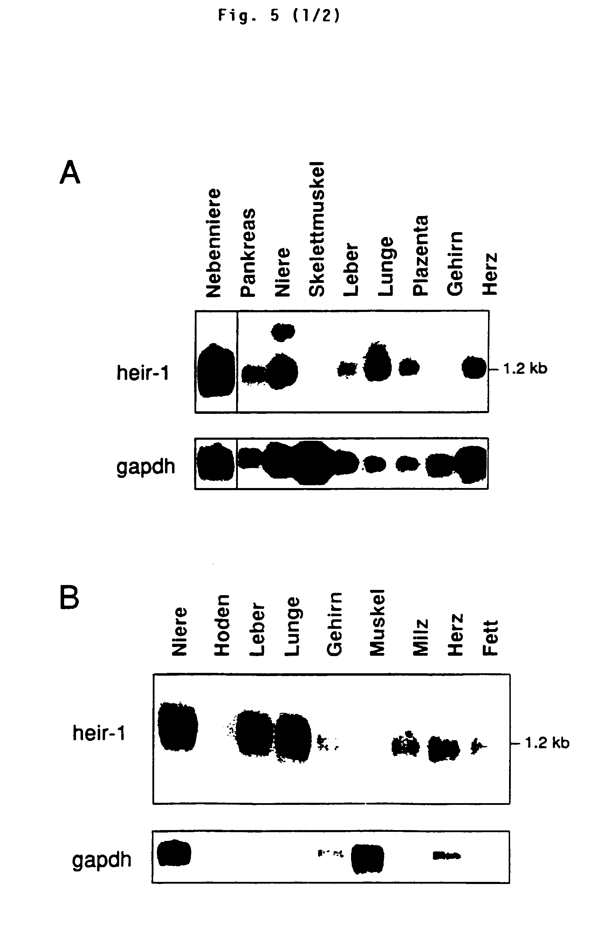

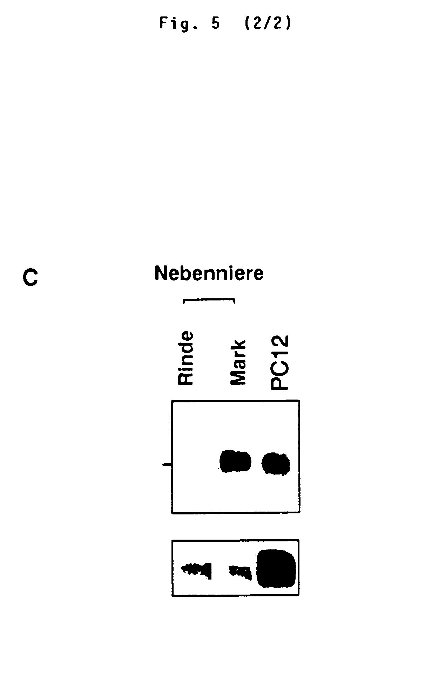

Um festzustellen, ob das HEIR-1-Gen gewebsspezifisch transkribiert wird, wurde eine Northern-Analyse durchgeführt. Die HEIR-1-cDNA wurde zuerst als Sonde verwendet, um Northern Blots, enthaltend poly(A)+RNA von neun verschiedenen adulten humanen Organen, zu untersuchen (Fig. 5A). Starke Signale wurden in RNA aus Lunge, Niere und Nebenniere erhalten; dies deutete darauf hin, daß das Gen in diesen Geweben in großen Mengen transkribiert wird. In Placenta, Muskel, Leber und Bauchspeicheldrüse war die Message nur in geringem Ausmaß vorhanden. Im Gehirn war ein Transkript des Gens nicht nachweisbar. Aus einer fötalen Gehirn-Library wurden zwei positive Klone erhalten. Die von einem der beiden Klone bestimmte Sequenz wich von der im Sequenzprotokoll angebenenen insofern ab, als diese Sequenz zusätzlich 100 Nukleotide aufwies. Da sich diese zusätzlichen Nukleotide zwischen zwei typischen Splice-Consensussequenzen befinden, dürfte sich der gefundene Klon von RNA ableiten, die nicht komplett prozessiert wurde; es ist jedoch nicht auszuschließen, daß diese Heterogenität eine funktionelle Relevanz aufweist. Unter den erfindungsgemäßen HEIR-1-Sequenzen gelten somit definitionsgemäß auch solche Sequenzen mitumfaßt, die sich von der im Sequenzprotokoll angegebenen durch zusätzliche Sequenzabschnitte unterscheiden, die auf alternatives Splicing zurückzuführen sind.

Die Nebenniere ist das primäre Zielorgan für Neuroblastom-Tumore. Mehr als 65 % der Tumore entwickeln sich aus Zellen des Nebennierenmarks(Pochedly, 1976; Russell und Rubinstein, 1989). Heir-1 Expression wurde in Markzellen untersucht, nachdem festgestellt worden war, daß heir-1 in der Nebenniere stark exprimiert wird. Zu diesem Zweck wurde die heir-1-Sonde mit Northern Blots, enthaltend poly(A)+RNA von Rindernebennierenrinde und - mark, hybridisiert. In Vorversuchen hatte sich in Zoo-Blot-Analysen gezeigt, daß heir-1 in allen Säugetierspezies hochkonserviert ist; das Rindergewebe erwies sich geeignet wie jedes andere Säugetiergewebe. Es wurde nachgewiesen, daß heir-1-Expression in der Nebenniere vorwiegend im Mark gefunden wird, während die Rinde fast kein Signal zeigt. Dieses Ergebnis wurde außerdem durch positive Signale auf kultivierten chromaffinen Zellen von Rattennebennierenmark (PC12-Zellen) bestätigt.

Aufgrund der hohen Homologie zwischen HEIR-1 und Maus-HLH462 und der leichteren Verfügbarkeit von Mausgewebe gegenüber Humangewebe wurde das Transkriptionsmuster der humanen Sonde in Mausgeweben untersucht, um ein Mausmodell für die gewebsspezifische Funktion des Gens im Menschen entwickeln zu können. Die Untersuchung eines Northern Blots von poly(A)+RNAs aus verschiedenen Mausgeweben zeigte hohe Transkriptkonzentrationen in der Lunge, Leber und Niere, während die Transkription in Fett, Herz, Milz, Gehirn und Muskel deutlich geringer war (Fig. 5B). Im Hoden konnte kein Transkript nachgewiesen werden. Die Transkriptionsmuster in den beiden verschiedenen Spezies waren somit nicht zur Gänze vergleichbar; insbesondere die unterschiedliche Expression in Leber zeigte, daß die beiden Organismen keine identische Verteilung des Genproduktes aufweisen.

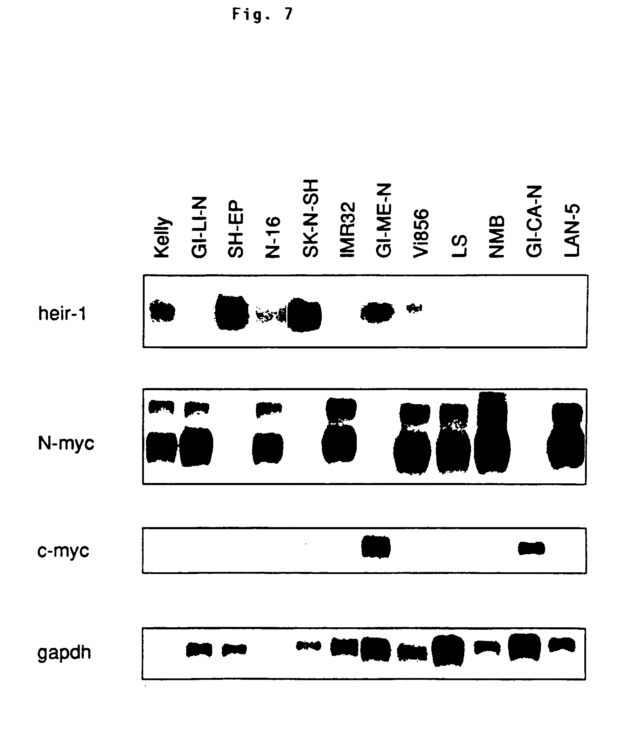

Nachdem gezeigt worden war, daß das HEIR-1-Gen in der Neuroblastom-Consensusdeletion lokalisiert ist, wurde die Transkription von HEIR-1 in Neuroblastomen untersucht. Northern Blots, enthaltend poly(A)+RNA von zwölf verschiedenen Neuroblastomzellinien, wurden mit der HEIR-1-cDNA als Sonde untersucht. Zu diesem Zweck wurde die hohe Expression des Gens in Nebennieren als normale Kontrolle angesehen, weil bekannt ist, daß mehr als 65% aller Neuroblastome in diesem Organ entstehen. Wie in Fig. 7 gezeigt wird, zeigten zehn der Tumor-RNAs sehr schwache HEIR-1-Signale, was auf eine spezifische Reduktion der Gen-Aktivität hindeutete. Starke Signale wurden nur in der RNA der Turmozellinie SK-N-SH und eines Subklons davon, SH-EP, gefunden. Trotz der beiden verschiedenen RNA-Konzentrationen zeigten alle Tumor-RNAs Banden derselben Größe, wie sie in normalen Geweben beobachtet wurden. Dies ist ein Hinweis darauf, daß die HEIR-1-mRNA von keinem turmorspezifischen strukturellen Rearrangement betroffen sein dürfte.

Die Amplifikation und Überexpression des Oncogens N-*myc* ist für viele Neuroblastome charakteristisch, besonders in fortgeschrittenen Stadien (Zimmerman und Alt, 1990). Um zu bestimmen, wie die beiden verschiedenen Niveaus der HEIR-1-Expression mit der Überexpression von N-*myc* in Zusammenhang stehen, wurde HEIR-1 von den Northern Blots abgelöst und die Blots erneut mit der Nb-1-Sonde, die spezifisch für das humane N-*myc*-Gen ist (Schwab et al., 1983), hybridisiert. Von den zwölf Tumorzellinien zeigten acht ein sehr starkes Signal, das auf Überexpression von N-*myc* hindeutete (Fig. 7). Beim Vergleich der HEIR-1-Transkription mit der Überexpression von N-*myc* in den jeweiligen Tumorzellinien zeigte sich ein deutlicher Zusammenhang: Diejenigen Tumorzellinien, die hohe Konzentrationen von N-myc zeigten, wiesen ein sehr niedriges Niveau von HEIR-1-mRNA auf. Bei den beiden Zellinien, bei denen dieser Zusammenhang nicht festgestellt wurde, ist die geringe HEIR-1-Transkription möglicherweise invers mit einer Überexpression von c-*myc* korreliert, wie die Re-Hybridisierung des Northern-Blots mit einer c-*myc*-spezifischen Sonde andeutet (s. Fig. 7); eine eindeutige Korrelation von HEIR-1 generell mit Genen der *myc*-Familie bedarf noch der definitiven Bestätigung.

Im Rahmen der vorliegenden Erfindung wurde weiters festgestellt, daß die Expression von heir-1 und N-myc einander auch in der Normalentwicklung ausschließen: Vom Proto-Oncogen N-myc ist bekannt, daß es in der normalen Embryonalentwicklung exprimiert wird (Zimmerman et al., 1986). Nachdem durch Isolation von cDNA-Klonen aus dem fötalen Gehirn erwiesen war, daß heir-1 in Embryonen exprimiert wird, wurde untersucht, ob eine inverse Korrelation von heir-1 und N-myc außer in Tumorzellen möglicherweise auch in der Normalentwicklung besteht. Zu diesem Zweck wurden beide Gene durch in situ Hybridisierung auf Mausgewebe in der Entwicklung analysiert und verglichen.

Die Vermutung, daß das HEIR-1-Gen in der Tumorgenese involviert ist, wurde somit durch die im Rahmen der vorliegenden Erfindung durchgeführten Untersuchungen der HEIR-1-Trenskription in normalen Geweben und in Neuroblastomzellen sowie durch die Beobachtung, daß die HEIR-l-Transkriptionslevels in humanen Neuroblastomen sowie in normalen Geweben während der Entwicklung invers mit einer Überexpression von N-myc korreliert sind, bestätigt.

Die vorliegende Erfindung betrifft in einem weiteren Aspekt die Verwendung von HEIR-1-DNA-Sequenzen oder Teilen davon sowie Antikörpern gegen HEIR-1 für die klinische Diagnose von pathologischen Zuständen, die mit einer Aberration im Neuroblastom-Consensus-Deletionsbereich lp36.2-p36.12 assoziiert sind bzw. zur Diagnose der Disposition zu solchen Erkrankungen. Unter solchen Erkrankungen sind in erster Linie Tumorerkrankungen zu verstehen, insbesondere Neuroblastome.

Daß die HEIR-1-cDNA einen RFLP in Apa I-verdauter genomischer DNA erkennt, der die Identifizierung von Chromosom 1-Homologen ermöglicht, stellt die Voraussetzung dafür dar, allelische Deletionen zu erkennen, die spezifisch für 1p36.2-p36.12 sind und z.B. in Neuroblastomen auftreten. Gegenüber bekannten RFLP-Sonden (Fong et al., 1989; Weith et al., 1989) ist die Information, die mit der erfindungsgemäßen DNA als Sonde erhalten wird, für die Neuroblastomdiagnose von größerer Aussagekraft, weil ein RFLP-Test auf Basis der erfindungsgemäßen Sonde ein Gen erfaßt, das direkt mit der Tumorgenese in Zusammenhang steht. Im Rahmen der durchgeführten Versuche wurde ein hoher Grad an Heterozygotie in Neuroblastompatienten festgestellt; aufgrund dieses hohen Heterozygotiegrades ist ein Test auf Basis der erfindungsgemäßen Sonde verläßlicher als die bekannten RFLP-Tests, die mit Sonden arbeiten, die einen geringeren Grad an Heterozygotie erfassen.

Im Zuge der durchgeführten RFLP-Tests zeigte sich, daß die Verwendung des kompletten kodierenden Bereichs der HEIR-1-cDNA verläßliche Ergebnisse bringt. Es sind jedoch neben der kompletten Sequenz auch Teile davon einsetzbar, die den RFLP erkennen. Die Eignung von cDNA-Fragmenten kann festgestellt werden, indem beliebige Fragmente, die im Hinblick auf ein detektierbares Signal zweckmäßig mindestens ca. 200 bp lang sind, bezüglich ihrer Erkennungsfähigkeit des RFLP mit der kompletten Sonde verglichen werden. Derartige Fragmente können z.B. mittels PCR-Amplifikation als Vorlage hergestellt werden.

Die erfindungsgemäße Verwendung der HEIR-1-cDNA-Sonden zur Diagnose von lp36.2-p36.12-Deletionen in Tumoren erfolgt mit Standardmethoden: Im Anschluß an die Entfernung eines Tumors wird die DNA, parallel dazu DNA aus gesundem Gewebe oder peripherem Blut des Patienten, präpariert, mit Apa I geschnitten und mittels Gelelektrophorese fraktioniert. Nach Southern-Transfer auf Membranen wird mit der erfindungsgemäßen Sonde hybridisiert (die Sonde muß nicht exakt mit der cDNA Sequenz übereinstimmen; es reicht aus, wenn sie in einem Ausmaß übereinstimmt, das unter den gewählten Bedingungen die Hybridisierung mit der zu analysierenden DNA, bzw. gegebenenfalls auch RNA, ermöglicht). Die Sonde kann mit beliebigen Markierungen versehen werden, die sichtbar gemacht werden können und die Hybridisierungsfähigkeit der Sonde nicht beeinträchtigen; Beispiele für gebräuchliche Markierungen sind Radioaktivität sowie Digoxigenin oder Biotin in Verbindung mit Fluoreszenz oder alkalischer Phosphatase-Reaktion.

Der RFLP-Test auf HEIR-1-Deletion wird zweckmäßig parallel zur Untersuchung auf Amplifikation des N-*myc*-Gens durchgeführt. Alternativ zu DNA kann RNA aus zu untersuchenden Geweben mit Hilfe der erfindungsgemäßen DNA analysiert werden, z.B. mit üblichen Northern Blots.

Zur Primärdiagnose von mit Aberrationen in der Region 1p36.2-p36.12 assoziierten pathologischen Zuständen sowie in jenen Fällen, in denen N-*myc*-Amplifikation und/oder HEIR-1-Deletion nicht oder nicht eindeutig nachweisbar sind werden Anti-HEIR-1-Antikörper eingesetzt. Die Assays unter Verwendung der Antikörper sind im allgemeinen herkömmliche Immonoassays, in denen die Probe auf Bildung eines binären oder ternären Komplexes zwischen dem HEIR-1-Genprodukt oder gegebenenfalls Fragment davon mit einem oder mehreren Antikörpern, von denen einer eine meßbare Markierung aufweist, bestimmt wird. Die Verwendung eines Antikörpers mit ausschließlicher Spezifität für HEIR-1, d.h. eines Antikörpers, der mit HEIR-1 verwandten Proteinen nicht kreuzreagiert, ermöglicht die spezifische Bestimmung von HEIR-1 in Geweben.

Aufgrund kürzlich durchgeführter Versuche (Bader et al., 1991) wird angenommen, daß der Chromosom lp-Arm ein Element enthält, das die Fähigkeit aufweist, den Tumor-Phänotyp zu einem nicht-transformierten Phänotyp zu revertieren. Da HEIR-1 in die in dieser Region gelegene Neuroblastom-Consensusdeletion kartiert, erfüllt es offensichtlich eine der wesetnlichen Voraussetzungen für ein Gen, das aufgrund seines funktionellen Verlustes an der Entstehung von Neuroblastomen beteiligt ist. Ein weiterer Hinweis dafür, daß HEIR-1 sowohl mit der Differenzierung als auch mit der Tumorgenese von Neuroblastomen in Zusammenhang steht, ergibt sich aus seinem Expressionsmuster: im Gegensatz zum Erwachsenengehirn wird es im embryonalen Gehirn transkribiert, was darauf hindeutet, daß das Gen in Geweben, die in der Entwicklung begriffen sind, aktiv ist und daher zu Differenzierungsprozessen beiträgt. Ein weiterer wesentlicher Aspekt der HEIR-1 Genaktivität, aufgrund dessen von HEIR-1 angenommen werden kann, daß es die Funktion eines Tumor-Suppressor-Gens hat, ist seine auffällige Transkription im Nebennierenmark im Gegensatz zu sehr geringer Transkription in den meisten Neuroblastomen. Diese Tatsache ist vor allem deshalb bemerkenswert, weil die Nebenniere das Zielgewebe der meisten Neuroblastome ist. Da allgemein anerkannt wird, daß Tumore im fortgeschrittenen Stadium und deren Metastasen vorwiegend aus Nebennierenmarkzellen stammen, läßt sich aus den vorhandenen Befunden schließen, daß die Aktivität von HEIR-1 in Tumoren spezifisch reduziert ist.

Sechs der untersuchten Neuroblastomzellinien waren auf ihre Fähigkeit zur Tumorbildung in vivo untersucht worden, fünf davon bildeten Tumore in immundefizienten Mäusen. Ein Vergleich der Tumorigenität mit der jeweiligen HEIR-1-Expression zeigt eine auffallende Übereinstimmung von normaler Genaktivität mit der Unfähigkeit, Tumore zu bilden. Daraus läßt sich schließen, daß HEIR-1 eine wesentliche Funktion zur Tumorunterdrückung in vivo hat.

Die nachgewiesenen Spuren von Transkripten, die in der Mehrzahl der Tumorzellinien gefunden wurden, resultieren vermutlich von der stark verminderten Expression der in den Tumorzellen verbleibenden Allele des Gens. Frühere zytogenetische und molekulare Untersuchungen legen nahe, daß wenigstens eine Kopie der Region, die heir-1 einschließt, in den verwendeten Tumorzellinien erhalten geblieben sein dürfte (z.B. GI-ME-N: Martinsson et al., 1989; N-16: Weith et al., 1989). Es ist zur Zeit nicht bekannt, ob für die geringen Mengen von heir-1 mRNA die Regulation der heir-1 Transkription oder posttranskriptionelle Änderungen verantwortlich sind. Um dies festzustellen, werden die 5'-Kontrollregionen des Gens untersucht.

Ein weiterer Hinweis für die Beteiligung von HEIR-1 bei der Neuroblastom-Tumorgenese ergibt sich aus dem Vergleich der HEIR-1-Transkription mit der N-*myc*-Expression in Neuroblastomen. Von N-*myc* ist bekannt, daß es spezifisch in einem hohen Anteil von Neuroblastomen im fortgeschrittenen Stadium überexprimiert wird und daß es positiv mit der Tumorprogression und Metastasenbildung korreliert ist. Folglich kann aus der inversen Korrelation zwischen HEIR-1- und N-*myc*-Aktivität eine funktionelle Rolle von HEIR-1 bei der malignen Transformation abgeleitet werden. Der Mechanismus der Korrelation ist noch nicht geklärt; aufgrund der vorliegenden Ergebnisse dürfte es jedoch am wahrscheinlichsten sein, daß die Reduktion der HEIR-1-Expression als tumorspezifisches Ereignis der N-*myc*-Überexpression vorausgeht und dieses Gen möglicherweise die Transkription von N-*myc* über einen noch unbekannten Weg negativ reguliert. Auch die Struktur des Gens, die charakteristisch für ein negativ reguliertes Gen ist, würde mit einem derartigen Mechanismus in Einklang stehen: ohne auf diese Theorie fixiert sein zu wollen, könnte durch die im gesunden Gewebe auftretende Dimerisierung von HEIR-1 mit einem potentiell als Oncogen wirkenden Genprodukt dieses inhibiert werden. Der Ausfall des HEIR-1-Gens könnte zur konstitutiven Aktivierung des Oncogens und damit zur Tumorinitiation führen. Einen weiteren Hinweis für eine negative Regulation gibt der gegenseitige Ausschluß der Aktivität der beiden Gene in sich entwickelnden Geweben, wobei das Ergebnis der Untersuchung des embryonalen Vorderhirns besonders aufschlußreich war: obwohl heir-1 und N-myc in demselben Entwicklungsstadium in demselben Gewebstyp, nämlich dem Neuroektoderm, exprimiert werden, sind sie in getrennten, doch einander eng benachbarten Bereichen aktiv. Der gegenseitige Ausschluß war besonders auffällig durch scharfe Grenzen zwischen den unterschiedlich exprimierenden Abschnitten.

Um die Funktion von HEIR-1 als Tumorsuppressor-Gen zu bestätigen, werden Versuche durchgeführt, mit Hilfe derer der Einfluß eines normal exprimierten HEIR-1-Gens (d.h. hinsichtlich Menge und Sequenz entsprechend dem in gesundem Normalgewebe exprimierten HEIR-1) in Tumorzellen beobachtet werden kann. Für solche Expressionsanalysen werden Tumorzellinien, z.B. die Neuroblastomzellinien IMR-32, Vi856 und SK-N-SH, mit in Säugetierzellen replizierbaren und selektionierbaren Vektoren, enthaltend die für HEIR-1 kodierende cDNA unter Kontrolle eines starken Promotors, transformiert und auf ihren Phänotyp, insbesondere ihre Malignität, untersucht. Der Verlust des malignen Phänotyps definiert das HEIR-1-Gen als Tumorsuppressor-Gen, mit Hilfe dessen pathologische Zustände therapeutisch behandelt werden können, die mit einer Fehlfunktion des Gens einhergehen. Dazu zählen vor allem diejenigen Tumorerkrankungen, die mit Hilfe der HEIR-1-Sonden diagnostiziert wurden. Dies sind in erster Linie Neuroblastome, in denen eine direkte Korrelation zwischen HEIR-1 und der Tumorgenese nachgewiesen wurde, sowie andere Tumore, die eine übereinstimmende Aberration im Chromosom lp36.2-p36.1 aufweisen (es gibt Hinweise dafür, daß andere Krebsformen signifikante Chromosomenaberrationen in derselben Region des Chromosoms 1 aufweisen wie Neuroblastome, z.B. Hepatome, maligne Melanome, Glioblastome, Merkel-Zell-Carcinome, Brustkrebs). Eine Bestätigung dafür, daß HEIR-1 an der Entstehung dieser Tumore beteiligt ist, kann erhalten werden, indem die für Neuroblastome mit HEIR-1 durchgeführten Untersuchungen, einschließlich der funktionellen Expressionsanalysen, in Zellen durchgeführt werden, die aus den interessierenden Tumoren stammen.

Das Prinzip einer therapeutischen Behandlung besteht darin, eine therapeutisch wirksame Menge an HEIR-1 dem Organismus zuzuführen oder im Organismus zur Expression zu bringen, also das Genprodukt oder im Rahmen einer Gentherapie das Gen dem Organismus zuzuführen, z.B. im Rahmen einer Ganzkörpertherapie. Das Gen als Bestandteil eines Vektors unter Kontrolle eines gewebsspezifischen Promotors wird unter Anwendung von Gentransfermaßnahmen, z.B. mit Hilfe von Liganden, die an gewebsspezifische Rezeptoren binden (eine solche Methode, die Transferrin-Konjugate mit DNA für die Rezeptor-vermittelte Endozytose benutzt, ist in der EP 388 758 beschrieben) in den Organismus eingebracht und im Zielgewebe zur Expression gebracht. Bei der Behandlung von Neuroblastomen eignen sich als Gentransfermaßnahmen Liganden für Rezeptoren, die in von der Neuralleiste abstammenden Zellen exprimiert werden, z.B. Transferrin (Versuche haben gezeigt, daß mit Transferrin assoziierte DNA effizient in solche Zellen aufgenommen wird). Nachdem die im Rahmen der vorliegenden Erfindung erhaltenen Transkriptionsmuster Gewebsspezifität der HEIR-1-Transkription gezeigt haben, wird im Rahmen einer Gentherapie grundsätzlich Gewebsspezifität angestrebt. Dies gilt auch im Fall der Verwendung retroviraler Vektoren, bei denen die Gewebsspezifität des verwendeten Genkonstruktes von großer Bedeutung ist, insbesondere aufgrund einer möglichen Dominanz des retroviralen Promotors. Zweckmäßig wird für Genkonstrukte zur Anwendung im Rahmen der Gentherapie der HEIR-1-Promotor verwendet, um dadurch möglichst Gewebsspezifität und Menge der HEIR-1-Expression entspechend der Expression im Normalgewebe zu erreichen. Um eine verstärkte Expression zu erreichen, können gegebenenfalls Promotor-Subfragmente in Mehrfachkopie in dem Konstrukt enthalten sein.

### Figurenübersicht

- Fig. 1:: Pulsfeld-Gelhybridisierung von Microklon pl-112B
- Fig. 2:: Restriktionskarte des Cosmids C1-112B
- Fig. 3:: cDNA-Sequenz (A) und abgeleitete Aminosäuresequenz von HEIR-1, Vergleich mit Maus-HLH462 (B)
- Fig. 4:: Aminosäure-Sequenzvergleich des HLH-Motifs zwischen HEIR-1, Maus-HLH462, Maus-Id und Drosophila emc. Die in allen vier Proteinen konservierten Aminosäuren sind eingerahmt.
- Fig. 5:: Gewebsspezifische Transkription des HEIR-1-Gens
- Fig. 6:: Lokalisierung des HEIR-1-Gens in der Neuroblastom-Consensus-Deletion
- Fig. 7:: Transkription von HEIR-1, N-myc und c-myc in Neuroblastom-Zellinien
- Fig. 8:: LOH-Analyse (Loss of Heterozygosity) von zwei Neuroblastom-Tumoren (N-15, N-29) mit der Sonde HEIR-1
- Fig. 9:: Western-Blot Analyse. Nachweis von rekombinantem HEIR-1 mittels polyklonalen Antikörpern

Die Erfindung wird anhand der folgenden Beispiele illustriert:

### Material und Methoden

### Zellinien und Neuroblastom-Patienten-Material

Folgende Neuroblastom-Zellinien wurden verwendet: Vi856; N15, N16 (Weith et al., 1989), SK-N-SH, SH-EP (Biedler et al., 1973), GI-ME-N (Donti et al., 1988), GI-LI-N, GI-CA-N (Longo et al., 1988), LS (Rudolph et al., 1991), Kelly (Schwab et al., 1983), IMR-32 (Tumilowicz et al., 1970), NMB (Balaban-Malenbaum and Gilbert, 1977), LAN-5 (z.B. Minth und Dixon, 1990). Alle Zellinien wurden in RPMI mit 10% FCS, 1% Penicillin/Streptomycin und 4 mM L-Glutamin gehalten.
Die hybriden (MausXHuman) Zellinien 8:4-BE1, 20-EA3, 20-DH8 und 20-AE2 wurden von Martinsson et al., 1989, beschrieben. Ratten PC 12 Zellen (Greene und Tischler, 1976) wurden in DMEM, enthaltend 15 % Pferdeserum, 1 % Penicillin/Streptomycin und 4 mM L-Glutamin, gezüchtet.
Tumorgewebe und peripheres Blut der Patienten mit der Bezeichnung N-15 und N-29 wurden von Weith et al. (1989) beschrieben.

### DNA-Sonden

p1-112B ist ein 2.5kbp langer genomischer DNA-Klon aus einer 1pter-p35-spezifischen Mikroklonbank (Martinsson et al., 1989). Der ursprüngliche Mikroklon wurde durch Klonierung von Eco RI-Fragmenten in den lambda-Vector NM1149 hergestellt. Für die weitere Verwendung erfolgte eine Umklonierung des Fragments in den Plasmid-Vektor Bluescript KS+ (Stratagene). p1-112B wurde durch Southern-Hybridisierung auf einer Serie von (MausXHuman) Hybridzellinien in die Region lpter-p36.12 kartiert. C1-112B repräsentiert einen Cosmid-Klon, der mit Hilfe der Probe p1-112B aus einer genomischen Cosmid-Bibliothek (siehe unten) isoliert wurde. Das Insert von C1-112B hat eine Länge von ca. 37 kbp (Fig. 2). C1-112B/9.ORI ist ein 9.0 kbp Eco RI-Subfragment aus dem Cosmid C1-112B (Fig. 2). Zur Herstellung dieses Klons wurden 8 µg C1-112B DNA mit dem Restriktionsenzym Eco RI (Boehringer Mannheim) nach Angabe des Herstellers verdaut, in einem 0.6 % Agarose-Gel durch Elektrophorese fraktioniert und die DNA durch Ethidiumbromid-Färbung dargestellt. Ein Agarosestück, das das 9.0 kbp-Subfragment des Cosmids enthielt, wurde ausgeschnitten und die enthaltene DNA mit Hilfe einer Phenol-Extraktion aus der Agarose isoliert. Eine DNA-Konzentrationsmessung der erhaltenen DNA wurde nicht vorgenommen. Die DNA wurde anschließend mit 50 ng Eco RI-verdauter, dephosphorylierter Plasmid DNA (Bluescript KS+) in 10 µl Volumen in Gegenwart von 1 Unit T4-DNA-Ligase (Boehringer Mannheim) und Ligationspuffer nach Angabe des Herstellers ligiert. Die Ligation erfolgte für 14 h bei 12°C. Anschließend wurde der gesamte Ligationsansatz zur Transformation von XL-1blue Wirtsbakterien (Stratagene) verwendet. Die Transformation erfolgte nach Standard-Protokoll (Sambrook et al., 1989). Nach Ausplattierung der Zellen auf LB-Amp (LB-Medium + Ampicillin [50 µg/ml])-Pletten und Übernacht-Bebrütung konnten rekombinante Kolonien isoliert werden. Nb-1 ist ein N-myc-spezifisches genomisches 1.0 kbp EcoRI/BamHI-Fragment, kloniert in pBR322 (Schwab et al., 1983). Der c-myc spezifische Klon pMc-myc 54 ist ein 1.3 kbp cDNA-Fragment, kloniert in pSP64 (Darveau et al., 1985).

### Radioaktive Markierung von DNA- und cDNA-Sonden

Für die Verwendung als Sonden zur Hybridisierung wurden Insert-Fragmente der jeweiligen rekombinanten Klone verwendet. Jeweils 5 bis 8 µg einer klonierten DNA wurden dazu mit Restriktionsenzymen zur Abtrennung des Inserts von der Vektor-DNA verdaut, durch Agarose-Gelektrophorese fraktioniert und das Insert-Fragment in einem Agarosestück herausgetrennt. Anschließend wurde die DNA durch Phenolisierung von der Agarose abgetrennt. 20-50 ng dieser Insert-DNA wurden durch "Random priming" nach Standard-Protokoll (Sambrook et al., 1989) mit ³²p-dCTP markiert und als Hybridisierungs-Proben eingesetzt.

### Analyse genomischer DNA

Genomische DNA aus Vollblut und diversen Organ- bzw. Gewebeproben wurde nach Standardprotokollen (Sambrook et al., 1989) isoliert, mit Restriktionsendonukleasen nach Angaben der Hersteller (Boehringer Mannheim, New England Biolabs, Promega) verdaut und durch Gelelektrophorese in 0.8 % Agarose-Gelen fraktioniert. Anschließend erfolgte partielle Depurinierung, Denaturierung und Kapillartransfer auf Nylon-Membranen (GeneScreen plus, NEN) unter denaturierenden Bedingungen entsprechend etablierten Protokollen (Sambrook et al., 1989). Die Membranen wurden nach dem Transfer der DNA luftgetrocknet, bei 80°C für 30 min inkubiert und UV-bestrahlt. Zur Hybridisierung wurden Membranen zunächst in 500 mM Na-Phosphatpuffer pH 7.2, 7 % SDS und 1 mM EDTA prähybridisiert (68°C, 1-2 h) und dann bei 68°C in dem gleichen Puffer mit der Probe übernacht unter ständiger Bewegung inkubiert. Die Membranen wurden dann generell stringent gewaschen (40 mM Na-Phosphatpuffer pH 7.2, 1 % SDS, 68°C), feucht in PE-Folie eingeschweißt und gegen Röntgenfilm (Kodak XAR-5) mit Verstärkerfolie exponiert.

### RNA-Analysen

Gesamt-RNA aus Geweben und aus Zellkultur-Material wurde mit der Guanidinium-Thiocyanat-Methode von Chomczynski und Sacchi (1987) isoliert. Poly(A)⁺-RNA wurde nach Standardmethode (Sambrook et al., 1989) mit Affinitätschromatographie durch Oligo-d(T)-Säulen isoliert. Poly(A)⁺-RNA wurde aus jeweils ca. 2-5 mg Gesamt-RNA isoliert. Die Ausbeute betrug ca. 2-3 % der Gesamt-RNA. Für die Northern-Analyse wurden aus jedem Gewebe 2 µg (normale menschliche Gewebe) oder 3 µg (Maus-Gewebe und Neuroblastom-Kulturzellen) in Formaldehyd-Agarose-Gelen nach einschlägigen Methoden (Sambrook et al., 1989) fraktioniert und auf Membranen (GeneScreen, NEN) transferiert. Die Hybridisierung der Northern blots erfogte nach gleichem Protokoll wie für Southern blots.

### DNA-Sequenzanalyse

Es wurde der Dyedideoxy-Sequenzierkit, erhältlich von Applied Biosystems, verwendet.

### Beispiel 1

### Pulsfeld-Gelelektrophorese

Hochmolekulare genomische DNA wurde aus humanen Spermien oder Lymphozyten, die in Agarose Blocks eingeschlossen worden waren, nach publizierten Methoden (Herrmann et al., 1987) isoliert. Die DNA in den Agarose Blocks (ca. 10 µg/block) wurden mit methylierungsempfindlichen, selten schneidenden Restriktions-Endonukleasen (2 bis 30 Units/Block) nach Empfehlung der Hersteller (New England Biolabs, Boehringer Mannheim) entweder in Einzel- oder in Doppelverdaus verdaut. Darauf wurden die Agarose-Blöcke mit der verdauten DNA in 1 % Agarose-Gelen durch CHEF-Elektrophorese im pulsierenden elektrischen Feld aufgetrennt. Dazu wurde ein Pulsaphor-System (LKB) verwendet. Die DNA-Auftrennung erfolgte in dreiphasigen Läufen: Phase 1: 40 s (8 h), Phase 2: 15 s (8 h), Phase 3: 3 s (8 h). Elektrophorese wurde in 0.5 x Tris-Borat-EDTA (TBE, siehe Sambrook et al., 1989) bei 200 V und 10°C durchgeführt. Anschließender Southern-Transfer und Hybridisierung von Membranen wurde wie oben beschrieben vorgenommen.
Das Ergebnis der PFGE ist in Fig. 1 dargestellt:
A: ³²P-markierter p1-112B wurde mit einem PFG-Blot hybridisiert, der ca. 10 µg normale humane Spermien-DNA in jeder Spur, verdaut mit den angegebenen Enzymen, enthielt. Das Fragment, das den Abstand zwischen den beiden CpG-Islands darstellt, ist mit einem Pfeil gekennzeichnet. Die Größen der lambda-DNA-Concatameren sind als Marker in kbp angegeben. Die Autoradiographie-Belichtung betrug drei Tage. B: Schematische Darstellung der genomischen Region, die durch pl-112B charakterisiert ist. Die Sonde ist durch einen schraffierten horizontalen Balken dargestellt. Zwei CpG-Inseln sind durch einen Cluster von senkrechten Balken angegeben. Zusätzliche Banden, die mit der Probe nachgewiesen wurden (z.B. Spuren NarI und SmaI in A, gezeichnet als vertikaler Balken in B) stammen von Erkennungsstellen innerhalb der Sonde und repräsentieren Fragmente außerhalb des Abschnittes zwischen den CpG-Islands.

### Beispiel 2

### a) Herstellung einer Cosmid-Bibliothek

Hochmolekulare genomische DNA wurde aus humanen Lymphzcyten isoliert: aus 40 ml peripherem Blut wurden die Erythrozyten lysiert und die DNA der Lymphozyten in 20 mM Tris-HCl, pH 7.6, 20 mM EDTA, 1 % Sarkosyl und 200 µg/ml Proteinase K (Boehringer Mannheim) extrahiert. Die erhaltene DNA wurde ohne vorhergehende Phenolisierung mit dem Restriktionsenzym Mbo I partiell verdaut, sodaß im Mittel 35-45 kbp große Fragmente entstanden. Eine Anreicherung von Fragmenten dieser Länge wurde durch die Gelfraktionierung in 0.25 % Agarose-Gelen erreicht.

Im Anschluß wurden die genomischen DNA Fragmente und Bam HI-geschnittene, dephosphorylierte Vektor-DNA (Cosmid-Vektor pWE15, Stratagene) im Gewichtsverhältnis 1:5 miteinander ligiert. Dazu wurden 5 µg genomische Fragmente und 25 µg Vektor-DNA in insgesamt 10 µl Volumen in Gegenwart von T4-DNA-Ligase und Ligationspuffer (Boehringer Mannheim) für 16 h bei 12°C inkubiert. Das ligierte Material wurde mit einem in-vitro packaging mix in infektiöse Phagenpartikel verpackt und zur Transformation von E. coli NM554-Zellen benutzt. Es wurde eine Ausbeute von 2x10⁷ Rekombinanten/µg genomischer DNA erzielt.

Je 2.5-2.8x10⁷ rekombinante Bakterien der Cosmid-Bibliothek wurden auf LB^{amp}-Platten der Größe 22x22 cm direkt auf Membranen (GeneScreen Plus, NEN) ausplattiert und für 13 h bebrütet. Von den primären Kolonie-Membranen ("Master-FIlter") wurden zwei Abdruck-Kopien ("Replika-Filter") hergestellt und erneut zum Koloniewachstum für ca. 8 h bebrütet. Die Master-Filter wurden anschließend mit Einfriermedium (LB + 20 % Glycerin) getränkt, auf Plexiglasplatten aufgelegt und bei -80°C gelagert. Replika-Filter wurden mit 0.2 M NaOH, 1 % SDS, 1.5 M NaCl für 5 min getränkt, in 50 mM Na-Phosphat-Puffer pH 6.5 neutralisiert und luftgetrocknet.

### b) Screening der Cosmid-Bibliothek mit der Sonde p1-112B

Insert-DNA aus pl-112B wurd durch random priming (s. oben) markiert und als Sonde auf die Replika-Filter der Cosmid-Bibliothek unter Standard-Bedingungen (Sambrook et al., 1989) hybridisiert. Areale mit positiven Kolonien wurden entsprechend aus den tiefgefrorenen Master-Filtern herausgeschnitten, in LB-Medium resuspendiert, und bei geringerer Dichte (ca. 600-1000 Kolonien/85 mm Filter) auf Filtern ausplattiert, bebrütet, und erneut mit markierter p1-112B-Sonde hybridisiert. Positive Signale konnten nach diesem Schritt Einzelkolonien zugeordnet werden. Die Einzelkolonien wurden isoliert und in 50 ml-Kulturen für die Isolation von Cosmid-DNA amplifiziert. Die Präparation von Cosmid-DNA erfolgte nach StandardMethoden (Sambrook et al., 1989).

### Beispiel 3

### Isolierung einer C1-112B/9.ORI positiven cDNA aus einer HeLa cDNA-library

140000 Plaques einer HeLa cDNA-library (Stratagene) wurden mit dem genomischen Subfragment C1-112B/9.ORI nach positiven cDNA-Plaques untersucht. Die prinzipiellen Arbeitsschritte einer Isolierung von cDNA Klonen aus einer cDNA-library werden im folgenden dargestellt.

### A. Primäres Screening:

1. Ausplattieren der Phagen: Die Phagen wurden entsprechend dem Ausgangstiter (1.5x10¹⁰ pfu/ml) mit TM verdünnt, sodaß pro 22x22 cm² Platte ungefähr 70000 Plaques vorhanden sind. Die entsprechende Menge an Phagen wurde dann mit 4 ml einer Übernachtkultur von XL-1 blue Bakterien für 20 min auf 37°C inkubiert. Danach wurden 40 ml Topagar (vorgewärmt auf 55°C) zu diesem Ansatz hinzugegeben und auf einer 22x22 cm2 großen LB-Platte ausplattiert. Anschließend erfolgte Inkubation bei 37°C übernacht.
2. Plaque-Transfer auf GeneScreen plus Membranen. Nach erfolgter Übernachtinkubation und anschließender Lagerung auf 4°C für 2 h wurde die Platte mit einer 21x21 cm² großen GeneScreen plus Membran für 2 min belegt. Dann wurde die Membran für 3 min auf ein mit Denaturierungslösung (1.5 M NaCl, 0.5 M NaOH) getränktes Filterpapier gelegt. Anschließend erfolgte eine 10 minütige Inkubation auf einem mit Neutralisierungslösung getränktes Filterpapier. Nach einstündiger Lufttrocknung und 30 minütiger Trocknung auf 80°C erfolgte eine Fixation der DNA auf der Membran mittels UV-Bestrahlung.
3. Hybridisierung der Membranen mit C1-112B/9.ORI Dieser Schritt erfolgte nach den gängigen Methoden (Sambrook et al., 1989).

### B. Sekundäres Screening:

Da Einzelplaques durch die hohe Dichte (70000 plaques/Platte) nicht erreicht werden konnten, mußte ein sekundäres Screening (mit dem Ziel der Isolierung von Einzelplaques) durchgeführt werden. 1. Isolierung von positiven Plaques Die Plaques, die im primären Screening positve Signale gegeben haben, wurden aus der großen Platte isoliert. Entsprechend dem in A angeführten Protokoll wurde ein sekundäres Screening durchgeführt. Die einzigen Unterschiede bei diesem Screening betrafen die Größe der Platten (8 cm Petrischalen) und die Dichte der Plaques/Schale (150 Plaques/Schale; verschiedene Verdünnungen des positiven Plaques wurden ausplattiert). 2. Analyse von positiven Einzelplaques Diese erfolgte nach den in Labors gängigen Methoden z.B. in-vivo Excision, durchgeführt nach den Vorschriften von Stratagene), PCR-analyse, etc.; Sambrook et al., 1989).

Es wurde der als HEIR-1 bezeichnete cDNA-Klon erhalten, der die in SEQ ID NO:1 dargestellte Sequenz aufweist. Die erhaltene cDNA-Sequenz ist auch in Fig. 3A abgebildet: Die beiden Startcodons, ein Polyadenylierungssignal und das Transkriptions-Terminationscodon sind unterstrichen, ein ATTTA-Motiv ist durch eine strichlierte Linie gekennzeichnet. Fig. 3B zeigt den Vergleich der abgeleiteten Aminosäuresequenz von HEIR-1 mit dem Maus-HLH462-Protein.
C. Direkte Sequenzierung der 5'-regulatorischen Region: Die Sequenz des Promotors wurde durch Sequenzierung des Klons C1-112B/90RI erhalten. Als Sequenzierprimer diente das in SEQ ID NO:6 dargestellte Oligonukleotid TGG GGA GTG AGT CCA GAG, welches zur Heir-1 cDNA komplementär ist.

### Beispiel 4

### Transkription des heir-1-Gens in verschiedenen Humangeweben

Das Ergebnis der Northern Hybridisierungen, durchgeführt, wie unter "Material und Methoden" angegeben, ist in Fig. 5 dargestellt.
Fig. 5A: Northern Blots von poly(A)⁺RNA (je 2 µg) aus neun verschiedenen adulten Geweben. Die linken acht Spuren stellen einen sog. MTN ("multiple tissue Northern") dar, erhältlich von Clontech (Palo Alto). Für die Isolierung von Nebennieren-RNA wurde Gewebe von einem Patienten mit einer Nieren- und Nebennieren-Operation verwendet. Die Größe der positiven Bande ist in kb angegeben. Die Kalibrierung der in jeder Spur aufgetragenen RNA-Menge wurde durch Hybridisierung einer Glycerinaldehyd-Phosphet-Dehydrogenase-Sonde (gapdh) mit dem Blot nach dem Abziehen der HEIR-1-Sonde vorgenommen (in der Fig. jeweils unten dargestellt). Die Autoradiographie-Belichtung betrug einen Tag (HEIR-1) bzw. 4 h (Kalibrierung).
Fig. 5B: Northern Blots von poly(A)⁺RNA (je 3 µg) von adulten Mausgeweben, hybridisiert mit humanem HEIR-1, wie unter A angegeben.
Fig. 5C: Northern Hybridisierungen der heir-1 cDNA-Sonde mit poly(A)+RNAs (je 3 µg) von Rindernebennierenrinde, rindernebennierenmark und Ratten-PC12-Zellen. Die Mengen von RNA wurden durch Methylenblaufärbung von RNA auf dem Filter und mit einer Ratten-GADPH-Sonde normalisiert. Die Größe der positiven Bande ist in kb angegeben. Die Autoradiographie wurde 2 1/2 Tage (heir-1 auf Rinder-RNA) bzw. 1 Tag (heir-1 auf PC12-Zellen) belichtet.

### Beispiel 5

### Transkription von heir-1 und c-myc in Neuroblastom-Zellinien

Northern Blots, enthaltend poly(A)+RNA (3 µg/Spur) aus zwölf verschiedenen Neuroblastom-Zellinien wurden hintereinander mit den drei angegebenen cDNA-Sonden hybridisiert. Die Kalibrierung wurde mit der GADPH-Sonde durchgeführt (unterste Spur). Die Autoradiographie-Belichtungen betrugen 5 bis 18 h (Fig. 7).

### Beispiel 6

### Inverse Korrelation der Expression von heir-1 und N-myc in embryonalem Mausgewebe

Die Präparation von embryonalen Gewebeschnitten und die in situ Hybridisierungen wurden durchgeführt, wie von Aguzzi et al., 1990, beschrieben. Sense- und Antisense-cRNA-Sonden wurden durch in vitro Transkription der heir-1 cDNA bzw. eines Maus-Subklons, der dem dritten Exon von N-myc entspricht (DePinho et al., 1986), in Gegenwart von 35S-markiertem rUTP hergestellt. Sensetranskribierte Sonden dienten als Kontrolle.

In situ Hybridisierung von Schnitten in Paraffin eingebetteter Mausgewebe aus verschiedenen Embryonalstadien, die entweder mit heir-1- oder N-myc-Antisense-Sonden hybridisiert wurden, zeigte für beide deutlich gewebsspezifische Signale. Insbesondere war die Expression jedes der Gene im sich entwickelnden Gehirn auf verschiedene Abschnitte des Neuroektoderms beschränkt. In der Schicht von Vorderhirn-Neuroektodermalzellen wurde N-myc vorwiegend in den Rinden- und Hypothalamusregionen exprimiert. Jedoch war ein scharf abgegrenzter ventraler Teil des Neuroektoderms, der das kaudale Telenzephalon und seine Verbindungen zu dem sich entwickelnden Dienzephalon umfaßt, vollständig frei von N-myc Message. Im Gegensatz dazu war dies der einzige Bereich des Vorderhirn-Neuroektoderms, der heir-1-Expression zeigte. Die Bereiche, die entweder heir-1 oder N-myc Expression aufwiesen, zeigten eine Trennung durch auffallend scharfe, im wesentlichen sich deckende Grenzen. Auch an der Grenze zwischen Neuroektoderm und Geweben mesodermalen Ursprungs wurde eine komplementäre Expression der beiden Gene festgestellt: heir-1 war stark exprimiert in den sich entwickelnden Schädelstrukturen, während N-myc in diesem Gewebe lediglich unspezifische Hintergrundwerte zeigte.

### Beispiel 7

### Identifikation eines Restriktions-Fragmentlängen-Polymorphismus (RFLP) für die Sonde HEIR-1

Genomische DNA wurde aus peripherem Blut von sieben verschiedenen Individuen nach Standardmethoden (Sambrook et al., 1989) isoliert. Die Kollektion von DNAs wurde mit vierzig verschiedenen Restriktions-Endonukleasen (Boehringer Mannheim, New England Biolabs, Promega) nach Maßgabe der Hersteller verdaut und durch Gelelektrophorese in 0.8 % Agarose-Gelen fraktioniert. Southern-Transfer der fraktionierten DNA auf GeneScreen Plus Membranen (NEN) erfolgte nach den oben beschriebenen Standardmethoden. Die Membranen wurden mit der ³²P-dCTP-markierten Sonde heir-1 nach den zitierten Standardmethoden hybridisiert, unter hohen Stringenzbedingungen (40 mM Na-Phosphatpuffer, pH 7.2, 1 % SDS, 68°C) gewaschen und für 24 h auf Kodak X-AR5 Film exponiert. Ein RFLP wurde durch den Vergleich der Hybridisierungsmuster auf DNAs erkannt, die mit jeweils dem gleichen Enzym verdaut worden waren. Das Bandenmuster auf Apa I-verdauten DNAs zeigte das Auftreten von zwei Allelen in verschiedenen Individuen. Größe der Allele, konstante Banden und Allelenfrequenz sind in Tabelle 1 zusammengefaßt:

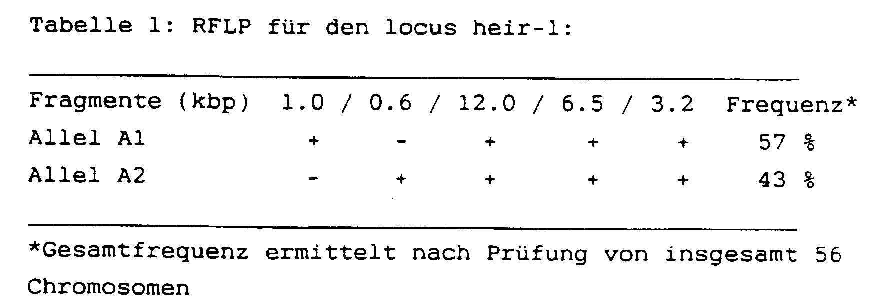

### Beispiel 8

### Lokalisierung von HEIR-1 in der Neuroblastom-Consensus-Deletion

Ein Southern Blot von EcoRI-verdauter DNA aus verschiedenen (MausXhuman)-Microcell-Hybriden und den jeweiligen humanen und Maus-Kontrollen wurde mit ³²P-markierter HEIR-1-cDNA hybridisiert. Der Southern Blot ist in Fig. 6 dargestellt.
A: Human (10 µg): Lymphozyten-DNA; Maus (20 µg): genomische Balb/c-DNA. Hybride (je 20 µg): 8:4-BE1, gesamtes humanes Chromosom 1; 20-EA3, del(1)(pter-p36.12); 20-DH8, del(1)(pter-p31); 20-AE2, del(1)(pter-p11). Ausgefüllte vertikale Balken in der unteren Fig. zeigen das Chromosom 1-Material, das in den Hybriden enthalten ist, an, schraffierte Regionen zeigen die jeweiligen Deletionen. die Sonde zeigte eine humane (H) und eine Maus (M)-Bande in den jeweiligen spuren.
B: Analyse des Verlustes der Heterozogotie (LOH) am HEIR-1-Lokus des Neuroblastompatienten N-29. Ein Southern Blot, enthaltend ApaI-verdaute normale DNA aus peripherem Blut (N) und Tumor(T)-DNA wurde mit der HEIR-1-Sonde hybridisiert. Normale DNA zeigt zwei Banden bei 1 kbp und 0.6 kbp, die den beiden HEIR-1-Allelen entsprechen. Die Tumor-DNA zeigt den Verlust des oberen Allels (Pfeil). Die Consensus-Deletion (cons.del.) ist als Bereich zwischen dem proximalen Bruchpunkt der Deletion in 20-EA3 und dem distalen Bruchpunkt der allelischen N-29-Deletion gezeigt. Die Autoradiographie-Belichtungen betrugen 16 h (A) bzw. 4 Tage (B).

### Beispiel 9

### Loss of Heterozygosity-Analyse in Neuroblastom-Tumoren

Jeweils 10 µg genomische DNA aus Tumor- und entsprechendem Normalgewebe (peripheres Blut) von Neuroblastom-Patienten, präpariert wie in Beispiel 1 beschrieben, wurde mit ApaI nach Vorschrift des Herstellers (Boehringer Mannheim) geschnitten, fraktioniert in 0.8 % Agarose-Gelen, auf Nylonmembranen transferiert und mit der ³²P-markierten HEIR-1-Sonde hybridisiert. Anschließend wurden die Filter, wie in Beispiel 1 beschrieben, stringent gewaschen und auf Röntgenfilm exponiert. Der RFLP ist in Fig. 8 dargestellt und zeigt das in Beispiel 7 beschriebene Allelen-Muster bei zwei repäsentativen NeuroblastomPatienten. Die bei N-15 in der Tumor-DNA sichtbare Resthybridisierung resultiert daraus, daß das zur Tumor-DNA-Isolierung verwendete Ausgangsmaterial Spuren von normalem Gewebe enthielt.

### Beispiel 10

### Expression von rekombinantem HEIR-1 in E.coli

Das von Studier et al., 1990, beschriebene E.coli Expressionsplasmid pET-2a wurde modifiziert, indem das kurze Ndel-BamHI Fragment durch ein 6 Histidin-Aminosäuren kodierendes Oligonukleotid ersetzt wurde (Adams et al., 1992). 3' zu diesem Oligonukleotid wurde unter Berücksichtigung des Leserahmens ein HEIR-1 kodierendes PCR Fragment, entsprechend der in SEQ ID NO:3 dargestellten Sequenz, kloniert. Dadurch enthält dieser bakterielle Expressionsvektor (pETH-2a-heir 1) Sequenzen, welche für ein (His)6x-HEIR-1 Fusionsprotein kodieren.
Nach Transformation des E.coli-Stammes BL21 (DE3) wurde das Protein entsprechend dem Standardprotokoll (Sambrook et al.) in E.coli durch Induktion mit IPTG exprimiert und nach Bakterienlyse mittels Nickel-Komplex-Affinitätschromatographie (Hochuli et al., 1988) aus dem bakteriellen Extrakt isoliert.

### Beispiel 11

### Herstellung von polyklonalen Antikörpern gegen HEIR-1

Zur Antikörpergewinnung wurde ein Kaninchen mit ungefähr 300 µg HEIR-1 Protein (emulgiert mit komplettem Freund's Adjuvans) immunisiert. Nach zwei und fünf Wochen wurde mit ungefähr 100 µg HEIR-1 Protein (emulgiert mit imkomplettem Freund's Adjuvans) zwecks Erhöhung der Antikörperkonzentration immunisiert ("boost").
Das aus dem immunisierten Kaninchen isolierte Serum wurde mittels Westernblot Analyse (Sambrook et al.), wie in Fig. 9 dargestellt, getestet. Die Detektion des proteingebundenen Antikörpers auf dem Westernblot erfolgte mittels ECL-Analyse (Enhanced Chemiluminescence, Amersham).
In Fig. 9 repräsentieren die mit 1 und 2 gekennzeichneten Bahnen des Blots gesamtes bakterielles Protein vor (1) bzw. nach (2) der Induktion mit IPTG. Die Bahn 3 enthält gereinigtes rekombinantes HEIR-1-Fusionsprotein.

### Literatur:

Adams, B., et al., 1992, Genes & Dev. 6, 1589-1607.
Aguzzi, A., Wagner, E.F., Williams, L.R. und Courtneidge, S.A., 1990, The New Biologist 2, 533-543.
Bader, S.A., Fasching, C., Brodeur, G.M. und Standbridge, E.J., 1991, Cell Growth and Diff. 2, 245-255.
Balaban-Malenbaum, G. und Gilbert, F., 1977, Science 198, 739-741.
Benezra, R., Davis, R.L., Lockshon, D., Turner, D.L. und Weintraub, H., 1990, Cell 61, 49-59.
Bernard, O., Cory, S., Gerondakis, S., Webb, E. und Adams, J.M., 1983, Embo J. 2, 2375-2383.
Biedler, J.L., Helson, L. und Spengler, B.A., 1973, Cancer Res. 33, 2643-2652.
Bird, A.P., 1986, Nature 321, 209-213.
Brawerman, G., 1989, Cell 57, 9-10.
Chomczynsky, P. und Sacchi, N., 1987, Anal. Biochem. 162, 156-159.
Christy, B.A., Sanders, L.K., Lau, L.F., Copeland, N.G., Jenkins, N.A. und Nathans, D., 1991, Proc.Nat.Acad.Sci. USA 88, 1815-1819.
Darveau, A., Pelletier J. und Sonnenberg, N., 1985, Proc.Nat.Acad.Sci. USA 82, 2315-2319.
Davis, R.L., Weintraub, H. und Lassar, A.B., 1987, Cell 51, 987-1000.
DePinho, R.A., Legouy, E., Feldman, L.B., Kohl, N.E., Yancopoulos, G.D. und Alt, F.W., 1986, Proc.Natl.Acad.Sci. USA 83, 1827-1831.
Donti, E., Longo, L., Tononi, G.P., Verdona, G., Melodia, A., Lanino, E. und Coraglia-Ferraris, P., 1988, Cancer Genet. Cytogenet. 30, 225-231.
Ellis, H.M., Spann, D.R. und Posakony, J.W., 1990, Cell 61, 27-38.
Fong, C.-T., Dracopoli, N.C., White, P.S., Merril, P.T., Griffith, R.C., Housman, D.E. und Brodeur, G.M., 1989, Proc.Natl.Acad.Sci. USA 86, 3753-3757.
Friend, S.H., Bernards, R. Rogelij, S., Weinberg, R.A. Papaport, J.M., Albert, D.M. und Dryja, T.P., 1986, Nature 323, 643-646.
Garell, J. und Modolell, J., 1990, Cell 61, 39-48.
Ghysen, A. und Dambly-Chaudiere, C., 1989, Trends in Genetics 5, 251-255.
Greene, L.A. und Tischler, A.S., 1976, Proc.Natl.Acad.Sci. USA 73, 2424-2428.
Harlow, E. und Lane, D., 1988, Antibodies, A Laboratory Manual, Cold Spring Harbor Laboratory.
Herrmann, B.G., Barlow, D.P. und Lehrach, H., 1987, Cell 48, 813-825.
Hochuli, E., Bannwarth, W., Döbeli, H., Gentz, R. und Stüber, D., 1988, Bio/Technology 6, 1321-1325.
Jones, N., 1990, Cell 61, 39-48.
Köhler, G. und Milstein, C., 1975, Nature 265, 495-497.
Longo, L., Christiansen, H., Christiansen, N.M., Cornaglia-Ferraris, P. und Lampert, F., 1988, J. Cancer Res. Clin. Oncol. 114, 636-640.
Lüscher, B. und Eisenmann, R.N., 1990, Genes Dev. 4, 2025-2035.
Martinsson, T., Weith, A., Cziepluch, C. und Schwab, M., 1989, Genes Chrom. Canc. 1, 67-78.
Minth, C.D. und Dixon, J.E., 1990, J. Biol. Chem. 265, 12933-12939.
Murre, C., Schonleber McCraw, P. und Baltimore, D., 1989a, Cell 56, 777-783.
Murre, C., Schonleber McCraw, P., Vaessin, H., Caudy, M., Lan, L.Y, Jan, Y.N., Cabrera, C.V., Buskin, J.N., Hauschka, S.D., Lassar, A.B., Weintraub, H. und Baltimore, D., 1989b, Cell 58, 537-5443.
Pochedly, C., 1976, In Pochedly, C. (ed.) Neuroblastoma, Edward Arnold Ltd., London, pp. 1-34.
Rudolph, G., Schilbach-Stückle, K., Handgretinger, R., Kaiser, P. und Hameister, H., 1991, Hum. Genet. 86, 562-566.
Russell, D.S. und Rubinstein, L.J., 1989, Pathology of Tumors of the Nervous System, 5th edition, Edward Arnold Publ., London, Melbourne, Auckland.
Sambrook, J., Fritsch, E.F. and Maniatis, T., 1989, Cold Spring Harbor Laboratory Press (2.Auflage).
Schwab, M., Alitalo, K., Klempnauer, K.H., Varmus, H.E., Bishop, J.M., Gilbert, F., Brodeur, G., Goldstein, M. und Trent, J., 1983, Nature 305, 245-248.
Studier, W., Rosenberg, A.H., Dunn, J.J. und Dubendorff, J.W., 1990, Methods Enzymol. 185, 60-89.
Tumilowicz, J.J., Nichols, W.W., Cholon, J.J. und Greene,A.E., 1970, Cancer Res. 30, 2110-2118.
Weinberg, R.A., 1991, Science 254, 1138-1146.
Weith, A., Martinsson, T., Cziepluch, C., Bruederlein, S., Amler, L.C., Berthold, F. und Schwab, M., 1989, Genes, Chrom. Canc. 1, 159-166.
Zimmerman, K.A., Yancopoulos, G.D., Collum, R.G., Smith, R.K., Kohl, N.E., Denis, K.A., Nau, M.N., Witte, O.N., Toran-Allerand, D., Gee, C.E., Minna, J.D. und Alt, F.W., 1986, Nature 319, 780-783.
Zimmerman, K. und Alt, F.W., 1990, Critical Reviews in Oncogenesis 2, 75-95.

## Patentansprüche

1. Isoliertes humanes Gen der Bezeichnung heir-1, enthaltend die in SEQ ID NO:3 angegebene DNA-Sequenz, die für ein Helix-Loop-Helix-Protein kodiert.

2. DNA nach Anspruch 1, dadurch gekennzeichnet, daß sie in 5'-Richtung vom Start-ATG die in SEQ ID NO:5 dargestellte Sequenz von Position 1 bis Position 443 enthält.

3. cDNA, abgeleitet von einem Transkript von DNA nach Anspruch 1, dadurch gekennzeichnet, daß sie die für HEIR-1 kodierende, in SEQ ID NO:3 dargestellte Sequenz enthält, einschließlich ihrer degenerierten Varianten, sowie Fragmente davon, die einen Apa I-Restriktionsfragment-Längenpolymorphismus erkennen.

4. Rekombinante DNA, dadurch gekennzeichnet, daß sie die in Anspruch 3 definierte cDNA, funktionell verbunden mit Expressionskontrollsequenzen, enthält, zur Expression in prokaryotischen oder eukaryotischen Wirtsorganismen.

5. Prokaryotische oder eukaryotische Wirtszellen, transformiert mit rekombinanter DNA nach Anspruch 4.

6. Rekombinantes HEIR-1 der in SEQ ID NO:4 dargestellten Sequenz, erhältlich durch Expression der in Anspruch 3 definierten cDNA.

7. Antikörper gegen HEIR-1.

8. Monoklonale Antikörper gegen HEIR-1.

9. Verfahren zur Diagnose von pathologischen Zuständen des Menschen, die mit einer Aberration der Chromosomenregion 1p36p12 assoziiert sind, dadurch gekennzeichnet, daß man eine Probe auf die Gegenwart von DNA gemäß Anspruch 1, die davon transkribierte RNA oder das exprimierte Genprodukt untersucht.

10. Verfahren nach Anspruch 9, dadurch gekennzeichnet, daß man aus dem zu untersuchenden Gewebe sowie aus gesundem Gewebe desselben Individuums DNA isoliert, die Proben mit Restriktionsenzym(en) verdaut und mit cDNA gemäß Anspruch 3 oder mit hinsichtlich ihrer Hybridisierungsfähigkeit unter den gewählten Bedingungen homologen Fragmenten davon hybridisiert.

11. Verfahren nach Anspruch 10, dadurch gekennzeichnet, daß man als Restriktionsenzym ApaI einsetzt.

## Claims

1. Isolated human gene designated heir-1, containing the DNA sequence specified in SEQ ID NO: 3, which codes for a helix-loop-helix protein.

2. DNA according to claim 1, characterised in that it contains the sequence shown in SEQ ID NO: 5 from position 1 to position 443, in the 5' direction from the start ATG.

3. cDNA derived from a transcript of DNA according to claim 1, characterised in that it contains the sequence shown in SEQ ID NO: 3 coding for HEIR-1, including the degenerate variants thereof and fragments thereof which recognise an Apa I restriction fragment length polymorphism.

4. Recombinant DNA, characterised in that it contains the cDNA defined in claim 3, functionally connected to expression control sequences, for expression in prokaryotic or eukaryotic host organisms.

5. Prokaryotic or eukaryotic host cells, transformed with recombinant DNA according to claim 4.

6. Recombinant HEIR-1 of the sequence shown in SEQ ID NO: 4, obtainable by expression of the cDNA defined in claim 3.

7. Antibodies against HEIR-1.

8. Monoclonal antibodies against HEIR-1.

9. Process for diagnosing pathological conditions in humans, associated with an aberration in chromosome region lp36p12, characterised in that a sample is investigated for the presence of DNA according to claim 1, the RNA transcribed thereby or the expressed gene product.

10. Process according to claim 9, characterised in that DNA is isolated from the tissue to be investigated and from healthy tissue from the same individual, the samples are digested with restriction enzyme(s) and hybridised with cDNA according to claim 3 or with fragments thereof which are homologous in terms of their hybridisation properties under the conditions selected.

11. Process according to claim 10, characterised in that ApaI is used as restriction enzyme.

## Revendications

1. Gène humain isolé d'appellation heir-1 contenant la séquence d'ADN indiquée dans SEQ ID NO : 3 qui code une protéine hélice-boucle-hélice.

2. ADN selon la revendication 1 caractérisé en ce qu'il contient dans la direction 5' à partir de l'ATG initiateur la séquence représentée dans SEQ ID NO : 5 de la position 1 à la position 443.

3. ADNc dérivé d'un transcrit d'un ADN selon la revendication 1 caractérisé en ce qu'il contient la séquence codant HEIR-1 représentée dans SEQ ID NO : 3 y compris ses variantes dégénérées, ainsi que des fragments de celles-ci, qui reconnaissent un polymorphisme de longueur de fragments de restriction Apa I.

4. ADN recombiné caractérisé en ce qu'il contient l'ADNc défini dans la revendication 3, lié de manière fonctionnelle à des séquences de contrôle de l'expression, pour l'expression dans des organismes hôtes procaryotes ou eucaryotes.

5. Cellules hôtes procaryotes ou eucaryotes transformées avec un ADN recombiné selon la revendication 4.

6. HEIR-1 recombinée ayant la séquence représentée dans SEQ ID NO : 4 qui peut être obtenue par l'expression de l'ADNc défini dans la revendication 3.

7. Anticorps contre HEIR-1.

8. Anticorps monoclonaux contre HEIR-1.

9. Procédé pour le diagnostic d'états pathologiques de l'être humain qui sont associés à une aberration de la région chromosomique Ip36p12, caractérisé en ce que l'on examine un échantillon concernant la présence d'ADN selon la revendication 1, de l'ARN transcrit à partir de celui-ci ou du produit génique exprimé.

10. Procédé selon la revendication 9 caractérisé en ce que l'on isole de l'ADN à partir du tissu à étudier et à partir de tissu sain du même individu, on digère les échantillons avec une ou des enzymes de restriction et on hybride avec un ADNc selon la revendication 3 ou avec des fragments de celui-ci homologues concernant leur capacité d'hybridation dans les conditions choisies.

11. Procédé selon la revendication 10 caractérisé en ce que l'on utilise ApaI comme enzyme de restriction.
